# EUROPÄISCHE PATENTSCHRIFT

(11) **EP 0 776 969 B1**
(45) Veröffentlichungstag und Bekanntmachung des Hinweises auf die Patenterteilung: **22.09.2004**
(21) Anmeldenummer: 96890172.8
(22) Anmeldetag: 19.11.1996
(51) Int. Cl.: C12N 9/00, C12P 21/06, C12N 9/10, C12N 9/64, C07K 14/755, C07K 14/815, C12N 9/74, C07K 17/00

(54) **Verfahren zur Herstellung von Proteinen durch kontrollierte proteolytische spaltung von Pro-Proteinen**
Process to prepare proteins by controlled proteolysis of pro-proteins
Procédé de préparation de protéines à travers une protéolyse controlée des pro-protéines

(30) Priorität: 24.11.1995 AT 192795
(43) Veröffentlichungstag der Anmeldung: 04.06.1997
(73) Patentinhaber: Baxter Aktiengesellschaft, 1221 Wien (AT)
(72) Erfinder: Fischer, Bernhard, Doz., 1160 Wien (AT); Mitterer, Artur, Dr., 2304 Orth/Donau (AT); Dorner, Friedrich, Prof., 1230 Wien (AT); Eibl, Johann, Dr., 1180 Wien (AT)
(74) Vertreter: Alge, Daniel, Mag. Dr. rer.nat.

(56) Entgegenhaltungen:
- EP-A- 0 367 489
- EP-A- 0 378 798
- EP-A- 0 416 890
- EP-A- 0 565 511
- FISCHER, B.E. ET AL.: "Immobilized Hirudin and Hirudin-based peptides used for the purification ..." PROTEIN EXPRESSION AND PURIFICATION, Bd. 8, 1996, Seiten 167-174, XP002096586

## Beschreibung

Die Erfindung betrifft ein Verfahren zur Herstellung und Gewinnung von Proteinen durch kontrollierte Spaltung von Pro-Proteinen mittels einer Protease.

Eine Vielzahl von Proteinen und Enzymen im lebenden Organismus wird durch die zelluläre Biosynthese als inaktive Vorstufen (Pro-Proteine (Vorläuferproteine) oder Pro-Enzyme) hergestellt. Diese inaktiven Vorstufen werden dann bei Bedarf in ihre aktiven Formen umgewandelt, z.B. durch limitierte Proteolyse. So wird im menschlichen Körper Prothrombin durch die Protease Faktor Xa in einer Prothrombinase-Komplex-Reaktion zu Thrombin umgewandelt. Inaktiver Faktor X wird z.B. durch die Protease Faktor IXa in aktiven Faktor Xa umgewandelt.

Die Gewinnung der aktivierten Proteine ist sowohl für die klinische Anwendung als auch für die Diagnostik von großem Interesse. Die aktivierten Proteine in reiner Form können dann z.B. zur Steuerung anderer proteolytischer Prozesse, wie der Blutgerinnung durch Thrombin in der Chirurgie, zur Therapie oder Diagnose oder zur Gewinnung von spezifischen Antikörpern verwendet werden.

Die aktiven Proteine können nur in sehr limitierter Menge aus lebenden Organismen, wie z.B. dem menschlichen Blut, gewonnen werden. Daher werden die Pro-Proteine oder Pro-Enzyme zumeist in vitro durch die Einwirkung geeigneter Proteasen in die aktivierten Formen umgewandelt. Ein derartiges Verfahren ist aus der EP-0 378 798 bekannt. Nach diesem Verfahren wird Prothrombin aus menschlichem Plasma an einen festen Träger adsorbiert und das Material mit Ca²⁺-Ionen behandelt und dadurch zusammen mit im Plasma vorhandenen Proteasen die Umwandlung von Prothrombin zu Thrombin bewirkt.

Ein anderes Verfahren ist in der EP-A-0 565 511 beschrieben, wo nach einer bevorzugten Ausführungsform das zu aktivierende Pro-Protein an einem Träger immobilisiert wird und dann durch eine lösliche Protease in das aktive Enzym umgewandelt wird. Um diese Umwandlung kontrollieren zu können, wird sie in Anwesenheit eines Detergens oder einer chaotropen Substanz durchgeführt. Zur Gewinnung der aktivierten Proteine sind dann jedoch weitere Reinigungsschritte notwendig, insbesondere um die Protease wieder abzutrennen.

Gemäß der EP-A-0 541 507 wird Prothrombin in löslicher Form durch Zugabe von gerinnungsaktiven Salzen zu einer Prothrombin-haltigen Lösung in Thrombin umgewandelt. Anschließend wird Thrombin durch Ionenaustauschchromatographie und/oder Affinitätschromatographie weiter gereinigt.

Gemäß der EP-A-0 565 512 wird Thrombin durch eine 150-minütige Behandlung einer Prothrombin-haltigen Lösung mit immobilisiertem Trypsin hergestellt. Die Verwendung der Protease in immobilisierter Form ermöglicht dabei eine leichtere Abtrennung der Protease nach der Aktivierung.

Gemäß der EP-A-0 416 890 wird rekombinantes humanes Protein C (rHPC) durch eine 2-stündige Behandlung mit an Glasperlen immobilisiertem Thrombin aktiviert. Die Trennung von aktiviertem rHPC von immobilisiertem Thrombin erfolgt durch Zentrifugation.

Es hat sich aber bei der Verwendung von Proteasen zur Aktivierung von Pro-Enzymen oder Pro-Proteinen das Problem gezeigt, daß der Prozeß der Proteolyse nicht auf der Stufe der aktiven Enzyme endet. Vielmehr werden durch die Protease im Verlaufe der Reaktion immer weitere Peptidbindungen hydrolysiert, und die aktivierten Proteine werden weiter zu wiederum inaktiven niedermolekularen Peptiden gespalten (Kisiel und Hanahan, 1973, Biochim. Biophys. Acta 329, 221-232). Daher ist die Ausbeute in solchen Verfahren bekanntermaßen gering, und es ergibt sich z.B. bei der Aktivierung von Prothrombin zu Thrombin durch Trypsin nur eine 50%-ige Ausbeute (Kisiel und Hanahan, 1973, Biochim. Biophys. Acta 329, 221-232).

Es wurde versucht, zur Verbesserung solcher Verfahren zur Aktivierungsreaktion Stabilisatoren, wie Albumin oder Glycin, zugegeben, um den weiteren Abbau der aktivierten Proteine zu verringern (Landaburu et al. 1961, Am. J. Physiol. 201, 298-302).

Ein wesentlicher Nachteil dieser Verfahren besteht jedoch darin, dass, wenn auch verlangsamt, ein weiterer Abbau zu Proteinfragmenten trotzdem stattfindet und Proteinmischungen entstehen, die zusätzlich große Mengen an Zusätzen, wie Detergentien oder Glycerin, enthalten. Es sind daher aufwendige Verfahren zur Reinigung der aktivierten Enzyme notwendig.

Proteinfragmente und Detergentien oder chaotrope Substanzen wurden gemäß der EP-A-0 565 511 mittels relativ aufwendigen chromatographischen Methoden entfernt.

In der EP 0 367 489 A werden thromboresistente Materialien beschrieben, bei welchen Hirudin oder Hirudin-Derivate kovalent an Trägermaterialien gebunden sind.

In der EP 0 416 890 A wird mittels immobilisiertem Thrombin aus Protein C aktiviertes Protein C generiert.

Die vorliegende Erfindung stellt sich daher zur Aufgabe, ein Herstellungsverfahren für Proteine aus Pro-Proteinen zur Verfügung zu stellen, mit dem eine hohe Ausbeute an Proteinen erzielt werden kann, und welches auf einfache Weise ermöglicht, Proteine mit sehr hoher spezifischer Aktivität und Reinheit zu gewinnen. Das Verfahren soll nicht nur auf bestimmte Ausgangslösungen beschränkt sein, sondern für ein umfassendes Spektrum an Ausgangslösungen anwendbar sein.

Diese Aufgabe wird erfindungsgemäß gelöst durch ein Verfahren zur Herstellung und Gewinnung von Proteinen durch kontrollierte proteolytische Spaltung von Pro-Proteinen mittels einer Protease, welches dadurch gekennzeichnet ist, dass eine Pro-Protein-haltige Lösung mit einer Protease und einem festen Träger, der eine höhere Affinität zum Protein als zum Pro-Protein oder des sen funktionell inaktiven Abbauprodukten aufweist, in Kontakt gebracht wird, wobei das Pro-Protein zum Protein proteolytisch gespalten wird und das Protein durch Adsorption an den festen Träger selektiv abgetrennt wird.

Bevorzugterweise wird eine immobilisierte Protease eingesetzt. Diese bietet den Vorteil, dass sie leicht aus der Lösung abgetrennt werden kann.

Die Pro-Protein-haltige Lösung wird mit der Protease während einer Kontaktdauer behandelt, die ausreicht, um eine Spaltung des Pro-Proteins zum Protein herbeizuführen, bei welcher jedoch im wesentlichen noch keine weiteren funktionell inaktiven Abbauprodukte des Proteins gebildet werden.

Es hat sich gezeigt, daß die Einwirkungsdauer der Protease auf das Pro-Protein ein wichtiger Faktor für die Qualität des herzustellenden Proteins ist, und die Einhaltung einer gegebenen Kontaktdauer zwischen Protease/Pro-Protein unter gegebenen Parametern für die Herstellung eines bestimmten Proteins wichtig ist. Die Kontaktdauer muß so gewählt sein, daß die Umwandlung vom Pro-Protein zum Protein erfolgt, daß jedoch ein weiterer, unerwünschter Abbau des Proteins nicht mehr erfolgen kann, sondern die Einwirkung der Protease vorher verhindert wird.

Kritische Parameter für die Ermittlung der optimalen Kontaktdauer sind daher die verwendete Protease, das Verhältnis von Pro-Protein zu Protease und die Reaktionstemperatur. Entsprechend dem erfindungsgemäßen Verfahren kann jeder Fachmann die kritischen Parameter sowie deren Optimierung ohne weiteres vor Reaktionsbeginn ermitteln, falls diese nicht ohnehin schon im Stand der Technik bekannt sein sollten.

Die für die Herstellung eines bestimmten Proteins spezifische Kontaktdauer kann jeweils durch einfaches Variieren der Kontaktdauer in getrennten Versuchsansätzen bestimmt werden. Die erhaltenen Produkte werden dabei jeweils analysiert und dann wird diejenige Kontaktdauer als gegebene Kontaktdauer definiert, mit der ein Maximum an erwünschtem Protein und ein Minimum an weiteren Abbauprodukten bzw. nicht gespaltenem Pro-Protein erhalten werden kann.

Diese gegebene Kontaktdauer ist selbstverständlich von System (Pro-Protein/Protease) zu System verschieden, sie liegt aber in vielen Systemen unter 24 Stunden. Als bevorzugte Einwirkungszeiten für die Protease haben sich Zeiträume zwischen 1 Sekunde und 24 Stunden, insbesondere zwischen 1 Sekunde und 5 Stunden, besonders bevorzugt zwischen 1 Sekunde und 30 Minuten erwiesen.

Als zusätzliche erfindungswesentliche Maßnahme wird, um die Kontaktdauer der Einwirkung der Protease möglichst exakt einzuhalten und den weiteren Abbau des Proteins zu verhindern, das durch die Protease gebildete Protein direkt nach der Proteasebehandlung an einen Träger gebunden, der eine höhere Affinität zum Protein als zum Pro-Protein oder dessen funktionell inaktiven Abbauprodukten aufweist. Damit wird das Protein dem weiteren Einwirken der im Reaktionsgemisch entzogen, und ein weiterer Abbau des Proteins kann nicht mehr stattfinden.

Dies kann beispielsweise ermöglicht werden, indem man den Affinitätsträger für das Protein in der Pro-Protein- bzw. Protease-haltigen Lösung vorsieht oder, indem man zwei chromatographische Säulen hintereinanderschaltet, wobei sich in der ersten Säule die (immobilisierte) Protease und in der zweiten der feste Träger für das Protein befindet, und das Eluat der ersten Säule direkt auf den Kopf der zweiten Säule kommt. Gemäß einer bevorzugten Ausführungsform des erfindungsgemäßen Verfahrens kann - für den Fall, daß die Proteasebehandlung nicht quantitativ erfolgt ist - das in der Lösung verbliebene nicht-gespaltene Pro-Protein in einem Recycling-Prozeß erneut mit der (immobilisierten) Protease in Kontakt gebracht, gespalten und das entstandene Protein an den selektiven Träger gebunden werden. Beispielsweise kann dies durch Verbinden des Auslaufs des zweiten, selektiven Trägers mit der ersten, Protease-gekoppelten Säule erfolgen, wobei das nicht-gebundene Pro-Protein erneut der Einwirkung der Protease zugänglich gemacht werden kann. Diese Schritte können solange wiederholt werden, bis im wesentlichen das gesamte Pro-Protein zu Protein gespalten worden ist.

Neben der bevorzugten kontinuierlichen Durchführung des (der) Proteasebehandlungsschritte(s) und des (der) Adsorptionsschritte(s) ist es natürlich auch möglich, das Verfahren diskontinuierlich durchzuführen, indem die (immobilisierte) Protease und der hochselektive Träger für das entstandene Protein sich in ein und demselben Behälter befinden, wobei man, wenn zwei verschiedene feste Träger zum Einsatz kommen, einen der beiden festen Träger selektiv entfernen kann (beispielsweise indem entweder Protease oder Protein an eine magnetische Oberfläche oder an ein entfernbares Insert gebunden wird).

Das erfindungsgemäße Verfahren kann selbstverständlich für alle Protease-vermittelten Umwandlungen von Pro-Proteinen zu Proteinen eingesetzt werden, bevorzugt wird es zur Herstellung von Enzymen aus Pro-Enzymen verwendet.

Zur proteolytischen Spaltung von Pro-Proteinen eignet sich eine Vielzahl von Proteasen, z.B. Chymotrypsin, Dispase, Endopeptidase Arg-C, Endoproteinase Lys-C, Endoproteinase Glu-C, Endoproteinase Asp-N, Faktor Xa, Kallikrein, Papain, Pepsin, Plasmin, Pronase, Proteinase K, Staphylocoagulase, Serin-Proteasen der Subtilisin-Familie, wie z.B. Kexin- oder Furin-artige Proteasen, oder Subtilisin, Thrombin, Trypsin (insbesondere humanes, bovines, porcines), trypsinartige Protease aus Arthropoden oder Mikroorganismen, wie z.B. Streptomyces griseus-Trypsin oder Serin-Proteasen aus Giftschlangen (Venom-Proteasen), wie z.B. die Prothrombin-Aktivatoren aus Echis carinatus Venom und aus Oxyuranus scutellatus Venom; die Aktivatoren für Faktor X und Faktor V aus Russell's Viper Venom, oder der Protein C-Aktivator Agkistrodon contortrix Venom. Bevorzugt eingesetzt werden als Protease Trypsin, Chymotrypsin, Kallikrein, Dispase, Endoproteinasen Glu-C, Lys-C oder Asp-N, Furin oder Faktor Xa. Es können selbstverständlich auch rekombinante Proteasen eingesetzt werden.

Die Protease wird - wie erwähnt - bevorzugterweise an einem festen Träger immobilisiert, sodaß zu keinem Zeitpunkt die Protease in löslicher Form mit dem Pro-Protein oder dem gebildeten Protein reagieren kann. Zur Immobilisierung der Protease eignen sich als natürliche oder synthetische Träger insbesondere Cellulose, Sepharose, Dextrane, insbesondere Agarose, Acrylate oder Silikate.

Gemäß einer routinemäßig verwendbaren bevorzugten Ausführungsform der Erfindung wird die immobilisierte Protease (das Protease-Gel) in eine Glassäule gepackt und die Pro-Protein-haltige Lösung durch das Protease-Gel filtriert. Die Fließgeschwindigkeit wird dabei so gewählt, daß die gegebene Kontaktdauer zwischen immobilisierter Protease und Pro-Protein eingehalten wird.

Das derart aktivierte Protein wird gemäß dieser Ausführungsform direkt aus dem Desorptionsüberstand (Eluat) an einen selektiven Träger adsorbiert. Im Hinblick auf eine gezielte Gewinnung des gewünschten Proteins mit hoher Reinheit und hoher Ausbeute sollte nicht aktiviertes Pro-Protein durch den spezifischen Träger möglichst nicht gebunden werden und die Säule, welche den selektiven Träger beinhaltet, ungehindert durchlaufen. Zur weiteren Aktivierung wird diese Pro-Protein-haltige Fraktion wiederum während der gegebenen Kontaktdauer mit dem Protease-Gel in Kontakt gebracht, worauf weiteres Protein gebildet wird, welches dann wiederum durch den selektiven Träger aus der Lösung adsorbiert wird. Durch die kontinuierliche, kontrolierte Aktivierung an Protease-Gel und Elution sowie die sofortige Adsorption des aktivierten Proteins an den selektiven zweiten Träger wird das Pro-Protein vollständig zu aktiviertem Protein umgewandelt, ohne daß aktiviertes Protein weiter durch die Protease gespalten wird. Aktiviertes Protein akkumuliert am selektiven Träger und kann anschließend vom Träger eluiert werden.

Besonders bevorzugte Proteine, die mit dem erfindungsgemäßen Verfahren hergestellt werden können, sind funktionell aktive Blutgerinnungsenzyme, welche aus den inaktiven Vorstufen dieser Enzyme durch proteolytischen Abbau hervorgehen. Beispielsweise können erfindungsgemäß Thrombin aus Prothrombin (Faktor II), Faktor IX aus pro-Faktor IX, Faktor IXa aus Faktor IX, von Willebrand-Faktor aus Pro-von Willebrand Faktor, Faktor Xa aus Faktor X, aktiviertes Protein C aus Protein C, Faktor XIIIa aus Faktor XIII, Faktor VIIa aus Faktor VII, Faktor VIIIa aus Faktor VIII, Faktor Va aus Faktor V, hergestellt werden.

Die Ausgangslösungen (Pro-Protein-haltige Lösungen) sind beim erfindungsgemäßen Verfahren auf keine besonders aufbereiteten Lösungen beschränkt. Bevorzugte Pro-Protein-haltige Lösungen enthalten aber das Pro-Protein in gereinigter Form, da dann eine besonders reine Protein-Präparation ermöglicht wird.

Pro-Protein-haltige Lösungen, welche das Pro-Protein in Mischung mit anderen Proteinen enthalten, sind jedoch ebenfalls für das erfindungsgemäße Verfahren geeignet, denn verunreinigende Proteine sind für das erfindungsgemäße Verfahren aufgrund seiner hohen Spezifität kein signifikanter Störfaktor.

Bevorzugterweise werden daher Pro-Protein-haltige Lösungen biologischen Ursprungs, insbesondere Plasma oder von Plasma oder von Plasmafraktionen abgeleitete Lösungen, im vorliegenden Verfahren eingesetzt.

Gemäß einer weiteren bevorzugten Ausführungsform des erfindungsgemäßen Verfahrens werden biotechnologisch hergestellte Pro-Protein-haltige Lösungen, insbesondere Kulturüberstände, welche von rekombinanten Zellkulturen erzeugt worden sind, als Ausgangslösungen eingesetzt.

Als für das herzustellende Protein selektive feste Träger zur Adsorption der aktivierten Proteine eignen sich besonders an einer Matrix immobilisiertes Heparin, immobilisiertes Benzamidin, immobilisiertes Hirudin oder von Hirudin abgeleitete Fragmente und Derivate, verschiedene immobilisierte Proteine oder Peptide, insbesondere spezifische immobilisierte Antikörper.

Von den Affinitätsträgern wird das Protein vorzugsweise selektiv eluiert, sodaß etwaige andere Proteine, die ebenfalls - wenn auch in geringen Mengen - an den Träger (spezifisch oder unspezifisch) gebunden werden sollten, abgetrennt werden.

Im Rahmen des erfindungsgemäßen Verfahrens können zur Inaktivierung eventuell vorhandener infektiöser Agentien, insbesondere humanpathogener Viren, zusätzliche allgemein aus dem Stand der Technik bekannte Virusinaktivierungs-Maßnahmen durchgeführt werden.

Gemäß einem anderen Aspekt betrifft die vorliegende Erfindung die Verwendung des erfindungsgemäßen Verfahrens zur Herstellung von Proteinen aus Pro-Protein, vorzugsweise von aktivierten Enzymen aus Pro-Enzymen, insbesondere die Herstellung von aktiviertem Faktor II, von aktiviertem Faktor V, von aktiviertem Faktor VII, von aktiviertem Faktor VIII, von Faktor IX, von aktiviertem Faktor IX, von aktiviertem Faktor X, von aktiviertem Faktor XIII, von von Willebrand-Faktor und von aktiviertem Protein C.

Die durch das erfindungsgemäße Verfahren erhaltene Protein-hal tige Fraktion eignet sich - aufgrund ihrer hohen Reinheit und ihrer hohen spezifischen Aktivität - in hervorragender Weise zur Herstellung einer pharmazeutischen Präparation und kann - aus gehend vom eluierten Protein - mit den üblichen Methoden zu einer solchen pharmazeutischen Präparation verarbeitet werden. Dabei können pharmazeutische Zusammensetzungen, welche ein gemäß dem erfindungsgemäßen Verfahren hergestelltes hochreines Protein, insbesondere Faktor IIa, Faktor Va, Faktor VIIa, Faktor VIIIa, Faktor IX, Faktor IXa, Faktor Xa, Faktor XIIIa, aktiviertes Protein C und/oder von Willebrand-Faktor, sowie gegebenenfalls einen oder mehrere phy siologisch akzeptable Träger und/oder andere pharmazeutische Zusatzstoffe enthalten, erhalten werden.

Die Wahl des selektiven festen Trägers für das gebildete Protein ist ein zentraler Punkt im erfindungsgemäßen Verfahren. Als besonders bevorzugte Träger haben sich immobilisiertes Hirudin und davon abgeleitete Polypeptide und Fragmente (insbesondere bei der Herstellung von Faktor IIa) erwiesen.

Dazu kann man Hirudin und davon abgeleitete Polypeptide und Peptid-Fragmente oder Derivate, welche an einer festen Oberfläche immobilisiert sind, verwenden. Als besonders geeignet erwiesen sich dabei solche von Hirudin abgeleitete Peptide, die vom C-terminalen Bereich des Proteins stammen und die Thrombin-Bindungsstelle beinhalten. Dabei werden die ausgewählten Peptide bevorzugt über Schwefel- oder Aminogruppen an den Träger gekoppelt.

Gemäß einer besonderen Ausführungsform der Erfindung werden so wohl gereinigter humaner Faktor II aus Plasma (Prothrombin, Stago Diagnostica), sowie gereinigter rekombinanter Faktor II (Falkner, F.G., et al.,Thromb. Haemost. (1992), 68, 119-124) eine Faktor II enthaltende Proteinmischung (Falkner, F.G., et al., Thromb. Haemost. (1992), 68, 119-124) und gereinigter Faktor X (Boehringer Mannheim) als Pro-Protein-haltige Lösung verwendet. Als Protease werden dabei bevorzugt sowohl immoilisiertes Trypsin, als auch immobilisierter Faktor Xa verwendet.

Dabei wird ein Protein-Komplex erhalten, enthaltend ein Protein, adsorbiert an einen festen Träger, hergestellt nach dem erfindungsgemäßen Verfahren, insbesondere einen Thrombinkomplex enthaltend Thrombin, welches an einen festen Träger adsorbiert ist, vorzugsweise an einen festen Träger enthaltend Hirudin oder von Hirudin abgeleitete Fragmente oder Derivate.

Zur Durchführung des erfindungsgemäßen Verfahrens kann eine Geräteanordnung, bestehend aus einem Behälter (I) enthaltend eine Protease, und einen Behälter (II) enthaltend einen festen Träger, wobei (I) und (II) direkt verbunden sind, verwendet werden. Diese Geräteanordnung wird vorzugsweise in dessen einstufiger Ausführungsform verwendet.

Bevorzugterweise ist Behälter (I) und (II) eine Säule, wobei das Endstück von (I) mit dem Kopfstück von (II) direkt gekoppelt ist und gegebenenfalls das Endstück von (II) mit dem Kopfstück von (I) verbunden ist.

Die Erfindung wird anhand der nachstehenden Beispiele und dazu gehörigen Zeichnungsfiguren, auf die sie jedoch nicht beschränkt sein soll, noch weiter erläutert.

Es zeigen:
Fig. 1 die elektrophoretische Analyse der Proteolyseprodukte von rekombinantem Faktor II (rFII) durch Trypsin gemäß Beispiel 1;
Fig. 2 die Abhängigkeit der Aktivität von entstandenem rFIIa in E/ml von der Fließgeschwindigkeit (ml/min);
Fig. 3-Fig. 11 die elektrophoretischen Resultate der Akti vierung von FII zu FIIa gemäß den Beispielen 2 bis 10; und
Fig. 12 die elektrophoretische Untersuchung der Aktivierung von FX zu FXa gemäß Beispiel 11.

### Beispiele:

### Aktivitätsbestimmungen:

Die Aktivitätsbestimmung des Faktors IIa (FIIa) erfolgte photometrisch in 50 mM Tris-HCl-Puffer, pH 8,0, 300 mM NaCl, 0,5% Albumin, 7,5 mM EDTA, bei 37°C. Als Substrat wurde das FIIa spezifische chromogene Substrat AcOH-H-D-CHG-Ala-Arg-pNA (erhältlich von der Firma Pentapharm) mit einer Konzentration von 0,2 mM verwendet. Durch enzymatische Hydrolyse aus dem Substrat freigesetztes para-Nitroanilin (pNA) wurde photometrisch in Abhängigkeit von der Zeit bei 405 nm bestimmt. Unter Verwendung eines FIIa-Konzentrationsstandards (Firma Immuno AG) wurde aus der Geschwindigkeit der Hydrolyse des Substrates die Aktivität der Probe ermittelt.

Die Aktivitätsbestimmung des Faktors Xa (FXa) erfolgte photometrisch in 50 mM Tris-HCl-Puffer, pH 7,8, 0,5% Albumin, bei 37°C. Als Substrat wurde das FXa-spezifische chromogene Substrat Bz-Ile-Glu(Piperidyl)-Gly-Arg-pNA (erhältlich von der Firma Chromogenix) mit einer Konzentration von 0,3 mM verwendet. Durch enzymatische Hydrolyse aus dem Substrat freigesetztes pNA wurde photometrisch in Abhängigkeit von der Zeit bei 405 nm bestimmt. Unter Verwendung eines FXa-Konzentrationsstandards (Firma Immuno AG) wurde aus der Geschwindigkeit der Hydrolyse des Substrates die Aktivität der Probe ermittelt.

Die Proteinbestimmung für natürlichen Faktor II und rekombinanten Faktor II (FII/rFII) bzw. FIIa/rFIIa erfolgte durch Messung der Absorption bei 280 nm unter Verwendung der Extinktionskoeffizienten (1%, 1 cm) von 13,8 bzw. 17,9 (Human Protein Data, Ed. by A. Haeberli, VCH Weinheim, New York, 1992).

Die Proteinbestimmung für FX bzw. FXa erfolgte unter Messung der Absorption bei 280 nm unter Verwendung eines Extinktionskoeffizienten (1%, 1 cm) von 12,4 (Human Protein Data, Ed. by A. Haeberli, VCH Weinheim, New York, 1992).

Die Bestimmung der Proteinkonzentrationen von Proteingemischen erfolgte mittels Bradford-Methode (M. Bradford, Anal. Biochem., 72 (1976), 248-254) unter Verwendung eines handelsüblichen Systems der Firma Bio-Rad.

### Beispiel 1

### Aktivierung von Prothrombin zu Thrombin durch immobilisiertes Trypsin in Abhängigkeit der Kontaktdauer zwischen Protease und Pro-Protein

4 ml rekombinantes Prothrombin (rekombinanter Faktor II, rFII) mit einer Konzentration von 0,5 mg/ml (Aktivität 4 I.E. FII/ml) in 20 mM Tris/HCl-Puffer, pH 8,0, 150 mM NaCl, wurde mit 0,1 ml immobilisiertem Trypsin-Agarosegel (Sigma, USA; 80 units Trypsin/ml Gel) versetzt und bei Raumtemperatur gerührt. Nach 1 Minute und nach 5 Minuten wurden Proben entnommen und auf Gehalt an rekombinantem Thrombin (rekombinanter Faktor IIa, rFIIa) untersucht (Daten siehe Tabelle 1).

**TABELLE 1**

| Probe | rFIIa-Aktivität (I.E./ml) |
|---|---|
| Ausgangsprobe | |
| rFII, 0,4 mg/ml, 4 ml | - |
| Trypsinverdau 1 Minute | |
| rFIIa, 0,4 mg/ml, 4 ml | 500 |
| Trypsinverdau 5 Minuten | |
| rFIIa, 0,4 mg/ml, 4 ml | 40 |

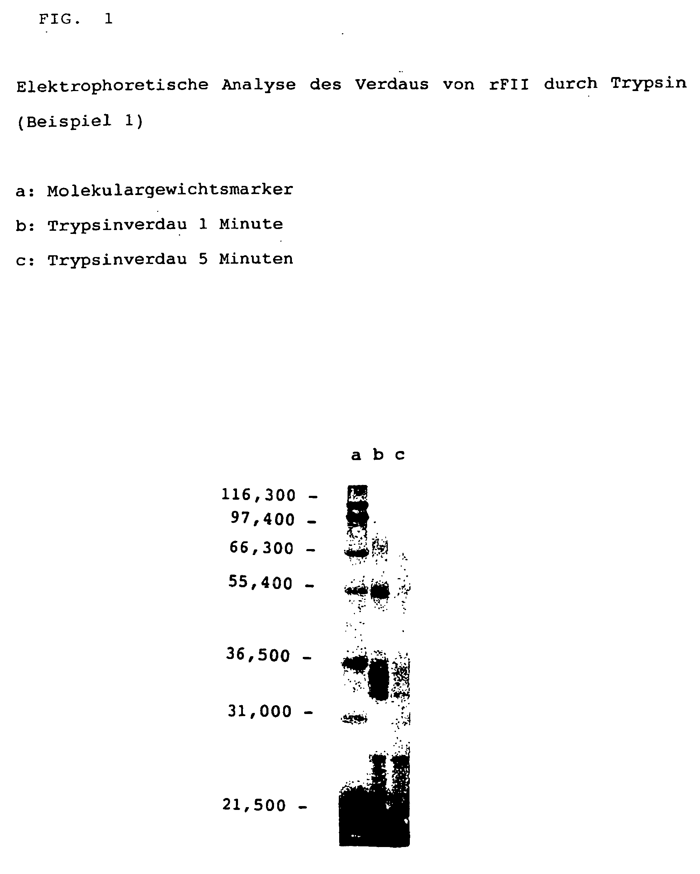

Die erhaltenen Proben wurden durch denaturierende Elektrophorese (Laemmli, Nature, Bd. 227:680-685, (1970)) auf ihre Proteinzusammensetzung untersucht. Die Ergebnisse sind in Fig. 1 dargestellt, wobei in Bahn a der Molekulargewichtsmarker, in Bahn b der 1-minütige Trypsinverdau und in Bahn c der 5-minütige Trypsinverdau dargestellt ist.

Aus diesen Ergebnissen geht hervor, daß durch den Verdau mit Trypsin rFII in rFIIa umgewandelt wurde. Nach nur 1 Minute Trypsineinwirkung auf rFII mit einer Aktivität von 4 I.E./ml wurde daraus rFIIa gebildet mit einer Aktivität von 500 I.E./ml. Bei einer Einwirkzeit von 5 Minuten jedoch war die nachweisbare Aktivität von rFIIa sehr gering. Die elektrophoretische Analyse zeigt, daß bei nur 1 Minute Trypsineinwirkung auf rFII ein Proteingemisch mit Molekulargewichten (in Da) zwischen 70000 und 20000 vorliegt, wobei ein Protein mit der Molekülmasse zwischen 31000 und 36000 dominiert. Da bekannterweise Thrombin in der Elektrophorese eine Molekularmasse von 33000 hat, kann angenommen werden, daß es sich bei dem Protein mit der Molekularmasse 31000 - 36000 um entstandenes Thrombin handelt. Jedoch sind auch noch Reste von nicht-aktiviertem rFII (Molekulargewicht 70000) vorhanden. Bei einer Inkubation von 5 Minuten von rFII mit Trypsin wurden dagegen nur nieder-molekulare Peptide in der Elektrophorese nachgewiesen. Das deutet auf einen nahezu vollständigen Verdau des rFII durch Trypsin hin, ohne daß jedoch rFIIa akkumulierte.

### Beispiel 2

### Aktivierung von rekombinantem Prothrombin zu Thrombin durch immobilisiertes Trypsin

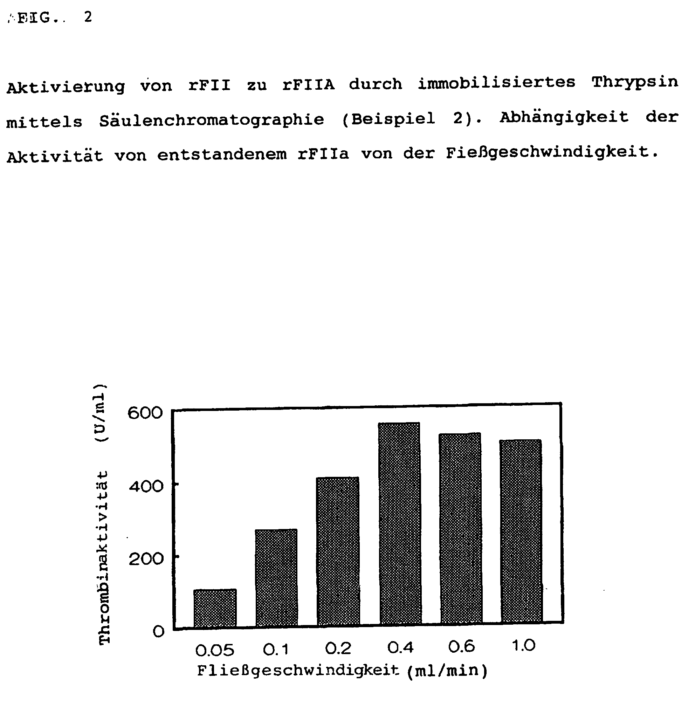

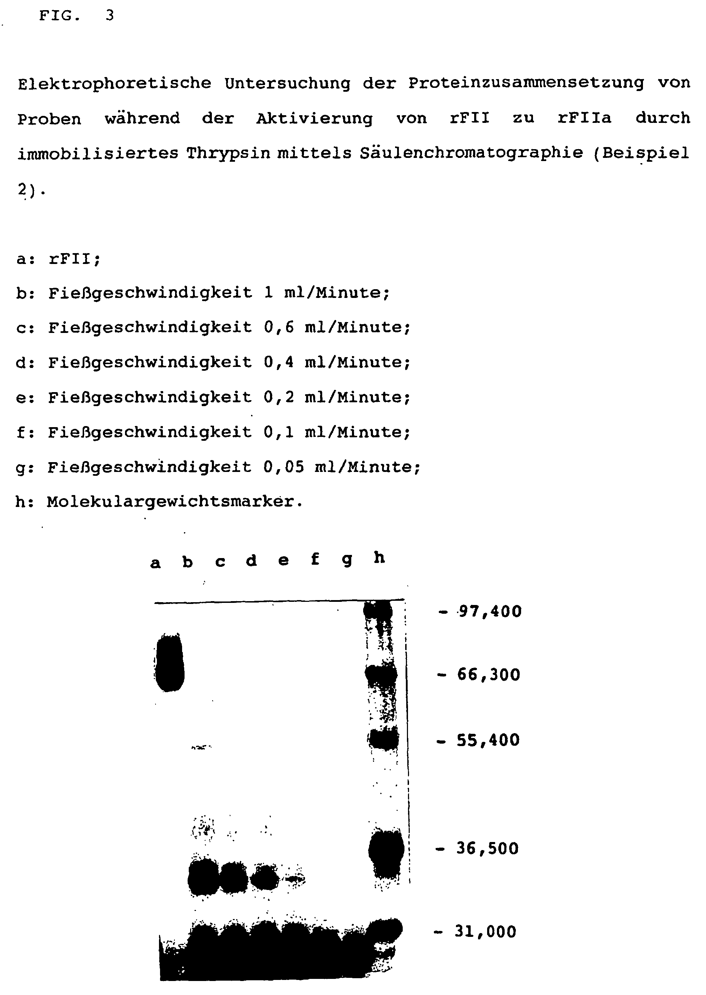

Eine Glassäule (Durchmesser 1 cm) wurde mit 0,1 ml immobilisiertem Trypsin-Agarosegel gefüllt; dies entspricht einer Gelhöhe von 1,25 mm. Durch dieses Trypsin-Agarosegel wurde mit unterschiedlichen Fließgeschwindigkeiten rFII gepumpt. rFII war zu 0,5 mg/ml (3,5 I.E./ml) in 20 mM Tris/HCl-Puffer, pH 8,0, 150 mM NaCl, gelöst. Die Fließgeschwindigkeit der rFII-Lösung durch das Gel wurde zwischen 0,05 ml/Minute und 1,0 ml/Minute variiert. Die einzelnen Eluate wurden auf ihren Gehalt an Thrombin-Aktivität (siehe Fig. 2) und auf ihre Proteinzusammensetzung mittels Elektrophorese (siehe Fig. 3) untersucht. Aus den Fließgeschwindigkeiten und der Dimension der Trypsin-Agarosegel-Säule (Volumen 0,1 ml Gel; Durchmesser der Säule 1 cm; Schichtdicke 1,25 mm) ergeben sich durchschnittliche Kontaktzeiten von 6 Sekunden, 10 Sekunden, 15 Sekunden, 30 Sekunden, 60 Sekunden und 120 Sekunden, bei entsprechenden Fließgeschwindigkeiten von 1,0 ml/Minute, 0,6 ml/Minute, 0,4 ml/Minute, 0,2 ml/Minute, 0,1 ml/Minute und 0,05 ml/Minute, zwischen rFII und dem immobilisiertem Trypsin.

Die in Fig.3 abgebildeten Elektrophoresebahnen sind mit den folgenden Proben beladen worden:
a: rFII;
b: Fließgeschwindigkeit 1 ml/Minute;
c: Fließgeschwindigkeit 0,6 ml/Minute;
d: Fließgeschwindigkeit 0,4 ml/Minute;
e: Fließgeschwindigkeit 0,2 ml/Minute;
f: Fließgeschwindigkeit 0,1 ml/Minute;
g: Fließgeschwindigkeit 0,05 ml/Minute;
h: Molekulargewichtsmarker.

Aus den Ergebnissen geht hervor, daß die Entstehung von rFIIa aus rFII durch Trypsinverdau sehr stark von der Fließgeschwindigkeit, d.h. von der Kontaktdauer zwischen rFII und immobilisiertem Trypsin abhängt. Die höchsten Aktivitäten an rFIIa wurden bei einer Fließgeschwindigkeit von 0,4 ml/Minute erzielt. Bei höheren Fließgeschwindigkeiten (0,6 ml/Minute und 1 ml/Minute) werden die Ausbeuten an rFIIa wieder geringer. Die Elektrophorese zeigt, daß bei diesen Fließgeschwindigkeiten nicht der gesamte rFII durch Trypsin proteolytisch in rFIIa umgewandelt wurde. Bei niedrigeren Fließgeschwindigkeiten (0,05 ml/Minute bis 0,2 ml/Minute) nimmt die Menge an rFIIa ebenfalls ab. Die elektrophoretische Untersuchung zeigt, daß durch die verlängerten Kontaktzeiten zwischen rFII und immobilisiertem Trypsin nur geringe Mengen an aktivem Thrombin akkumulieren, und mit abnehmender Fließgeschwindigkeit inaktive, nieder-molekulare Peptide entstehen.

### Beispiel 3

### Aktivierung von rekombinantem Prothrombin zu Thrombin durch immobilisiertes Trypsin und Gewinnung des Thrombins durch Affinitätschromatographie an Hirudin-Thiol-Sepharose.

4000 Anti-Thrombin units (ATU) Hirudin (erhältlich von der Firma Pentapharm) wurden reduziert und anschließend entsprechend der Herstellervorschrift an 1 ml aktivierte Thiol-Sepharose (Pharmacia) gekoppelt. Hirudin-Thiol-Sepharose (HTS) wurde in eine Glassäule (Durchmesser 1 cm) gefüllt.

Der Auslauf einer Glassäule (Durchmesser 1 cm) die 0,1 ml immobilisierte Trypsin-Agarosegel (TAG) enthielt, wurde durch eine direkte Schlauchverbindung mit dem Einlauf der HTS-Säule verbunden. Über ein Ventil und eine Pumpe konnte der Auslauf der HTS-Säule mit dem Einlauf der TAG-Säule verbunden werden, wodurch ein Flüssigkeitskreislauf entsteht. Beide Säulen wurden mit 20 mM Tris/-HCl-Puffer, pH 8,0, equilibriert.

rFII wurde zu 0,4 mg/ml (Aktivität 3,5 I.E./ml) in 4 ml 20 mM Tris/HCl-Puffer, pH 8,0, gelöst und mit einer Fließgeschwindigkeit von 0,8 ml/min durch die TAG-Säule gepumpt. Von dort wurde der Flüssigkeitsstrom ohne Unterbrechung direkt auf die HTS-Säule geführt und nach der Passage der HTS-Säule ein zweites Mal mittels Pumpe wiederum durch die TAG-Säule und HTS-Säule gepumpt. Anschließend wurde die HTS-Säule von der TAG-Säule getrennt, mit 50 mM Citratpuffer, pH 6,5, gespült (um nicht-gebundenes Material zu entfernen) und dann mit 50 mM Na-Citratpuffer, pH 6,5, 500 mM NaCl, gewaschen (0,5 M NaCl-Eluat). Anschließend wurde die HTS-Säule mit 1,5 M KSCN in 50 mM Citratpuffer, pH 6,5, eluiert (1,5 M KSCN-Eluat).

Die während der Aktivierung erhaltenen Fraktionen wurden auf Thrombinaktivität (I.E./ml), Gesamtaktivität (I.E.) und spezifische Aktivität (Thrombinaktivität/mg Protein) untersucht. In Tabelle 2 sind die Ergebnisse der rFII-Aktivierung aus Beispiel 3 zusammengestellt.

**TABELLE 2**

| Probe | rFIIa Aktivität | | rFIIa spezifische Aktivität |
|---|---|---|---|
| | (I.E./ml) | (I.E. gesamt) | (I.E./mg Protein) |
| Ausgangmaterial | 0 | 0 | 0 |
| Nicht-gebundenes Material | 0 | 0 | 0 |
| 0,5 M NaCl-Eluat | 50 | 100 | 300 |
| 1,5 M KSCN-Eluat | 390 | 2150 | 3400 |

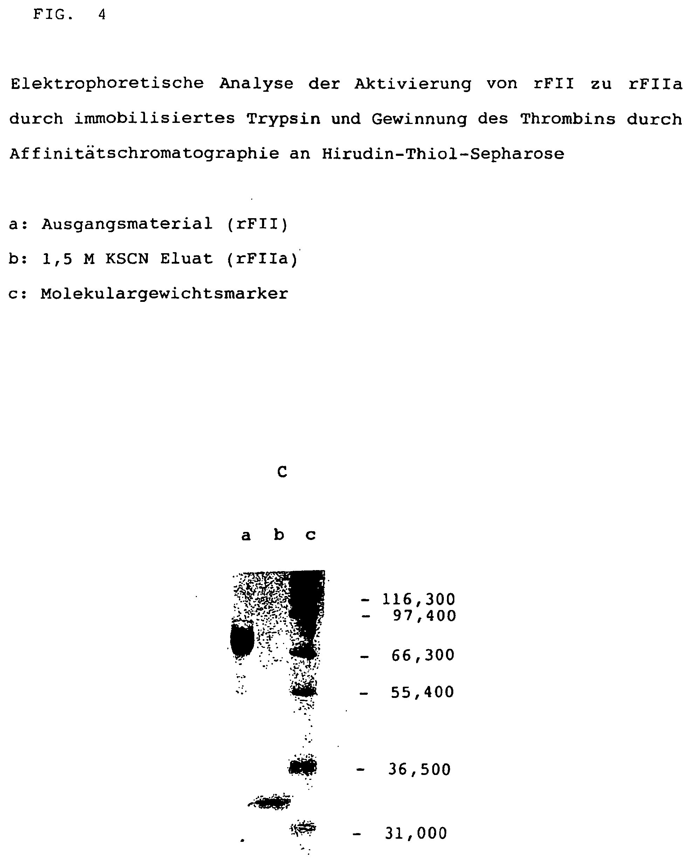

Fig.4 zeigt die elektrophoretische Untersuchung der beschriebenen Aktivierung, wobei in Bahn a das Ausgangsmaterial (rFII), in Bahn b das 1,5 M KSCN-Eluat (rFIIa)und in Bahn c ein Molekulargewichtsmarker aufgetrennt worden sind.

Die Ergebnisse zeigen, daß durch das in Beispiel 3 beschriebene Verfahren rFII effektiv in rFIIa umgewandelt wurde. Gebildeter rFIIa akkumulierte an der HTS-Säule und wurde in elektrophoretisch reiner Form mit einem Molekulargewicht von 33000 und mit einer sehr hohen spezifischen Aktivität durch spezifische Elution der HTS-Säule erhalten. Durch das beschriebene Verfahren konnten aus 1 I.E. rFII mehr als 150 I.E. reiner rFIIa gewonnen werden. Dies entspricht einer im wesentlichen quantitativen Umwandlung.

### Beispiel 4

### Aktivierung von rekombinantem Prothrombin zu Thrombin durch immobilisiertes Trypsin und Gewinnung des Thrombins durch Affinitätschromatographie an Thiol-Peptid-Thiol-Sepharose.

20 mg eines Peptides (Thiol-Peptid, TP) mit der Aminosäuresequenz NH2-Cys-Lys-Pro-Gln-Ser-His-Asn-Asp-Gly-Asp-Phe-Glu-Glu-Ile-Pro-Glu-Glu-Tyr-Leu-Gln-COOH wurden entsprechend der Herstellervorschrift an 1 ml aktivierte Thiol-Sepharose (erhältlich von der Firma Pharmacia) gekoppelt. Thiol-Peptid-Thiol-Sepharose (TPTS) wurde in eine Glassäule (Durchmesser 1 cm) gefüllt. Der Auslauf einer Glassäule (Durchmesser 1 cm) die 0,1 ml immobilisierte Trypsin-Agarosegel (TAG) enthielt, wurde durch eine direkte Schlauchverbindung mit dem Einlauf der TPTS-Säule verbunden. Über ein Ventil und eine Pumpe konnte der Auslauf der TPTS-Säule mit dem Einlauf der TAG-Säule verbunden werden, wodurch ein Flüssigkeitskreislauf entsteht. Beide Säulen wurden mit 20 mM Tris/HCl-Puffer, pH 8,0, equilibriert.

rFII wurde zu 0,4 mg/ml (Aktivität 3,5 I.E./ml) in 4 ml 20 mM Tris/HCl-Puffer, pH 8,0, gelöst und mit einer Fließgeschwindigkeit von 0,8 ml/min durch die TAG-Säule gepumpt. Von dort wurde der Flüssigkeitsstrom ohne Unterbrechung direkt auf die TPTS-Säule geführt und nach der Passage der TPTS-Säule ein zweites Mal mittels Pumpe wiederum durch die TAG-Säule und TPTS-Säule gepumpt. Anschließend wurde die TPTS-Säule von der TAG-Säule getrennt, mit 50 mM Citratpuffer, pH 6,5, gespült (um nicht-gebundenes Material zu entfernen) und dann mit 50 mM Na-Citratpuffer pH 6,5, 500 mM NaCl, gewaschen (0,5 M NaCl-Eluat). Anschließend wurde die TPTS-Säule mit 1,5 M KSCN in 50 mM Citratpuffer, pH 6,5, eluiert (1,5 M KSCN-Eluat). Die während der Aktivierung erhaltenen Fraktionen wurden auf Thrombinaktivität (I.E./ml), Gesamtaktivität (I.E.) und spezifische Aktivität (Thrombin-Aktivität/mg Protein) untersucht. Die erhaltenen Ergebnisse der rFII-Aktivierung sind in Tabelle 3 aufgelistet.

**TABELLE 3**

| Probe | rFIIa Aktivität | | rFIIa spezifische Aktivität |
|---|---|---|---|
| | (I.E./ml) | (I.E. gesamt) | (I.E./mg Protein) |
| Ausgangmaterial | 0 | 0 | 0 |
| Nicht-gebundenes Material | 0 | 0 | 0 |
| 0,5 M NaCl-Eluat | 45 | 100 | 300 |
| 1,5 M KSCN-Eluat | 380 | 2250 | 3250 |

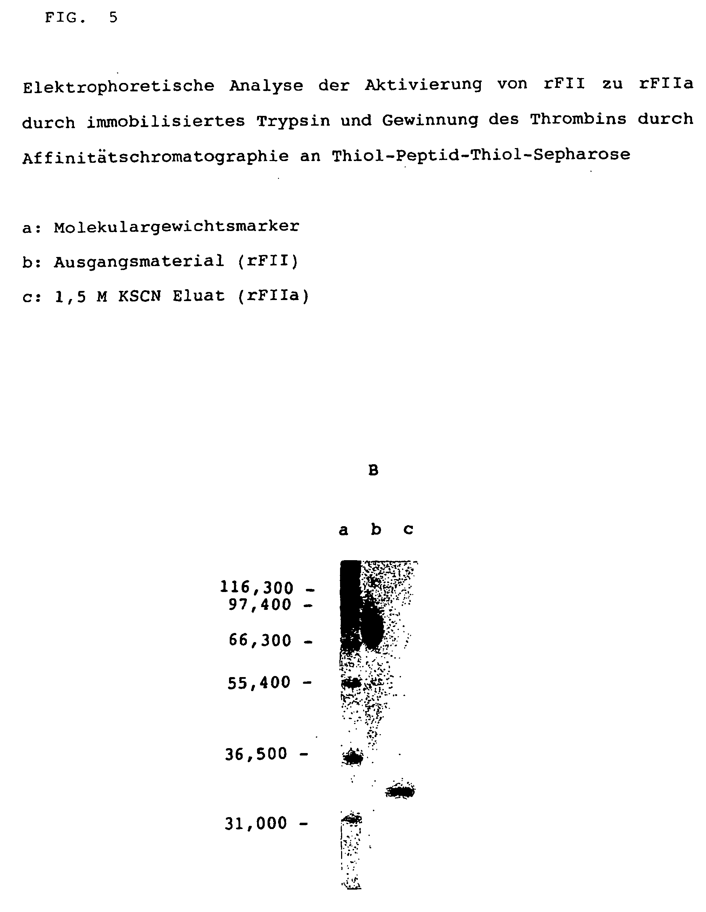

Die elektrophoretische Analyse der Aktivierung von rFII zu rFIIa durch immobilisiertes Trypsin und Gewinnung des Thrombins durch Affinitätschromatographie an Thiol-Peptid-Thiol-Sepharose ist in Fig.5 abgebildet, wobei in Bahn a der Molekulargewichtsmarker, in Bahn b das Ausgangsmaterial (rFII) und in Bahn c das 1,5 M KSCN-Eluat (rFIIa) aufgetragen worden sind.

Die Ergebnisse zeigen, daß durch das beschriebene Verfahren rFII effektiv in rFIIa umgewandelt wurde. Gebildeter rFIIa akkumulierte an der TPTS-Säule und wurde in elektrophoretisch reiner Form mit einem Molekulargewicht von 33000 und mit sehr hoher spezifischer Aktivität durch spezifische Elution der TPTS-Säule erhalten. Durch das beschriebene Verfahren konnten aus 1 I.E. rFII mehr als 150 I.E. reiner rFIIa gewonnen werden.

### Beispiel 5

### Aktivierung von rekombinantem Prothrombin zu Thrombin durch immobilisiertes Trypsin und Gewinnung des Thrombins durch Affinitätschromatographie an Amino-Peptid-CH-Sepharose.

20 mg eines Peptides (Amino-Peptid, AP) mit der Aminosäuresequenz NH2-Lys-Pro-Gly-Pro-Gly-Ser-His-Ala-Asp-Gly-Asp-Phe-Glu-Glu-Ile-Pro-Glu-Glu-Tyr-Leu-COOH wurden entsprechend der Herstellervorschrift an 1 ml aktivierte CH-Sepharose (Pharmacia) gekoppelt. Amin-Peptid-CH-Sepharose (APCHS) wurde in eine Glassäule (Durchmesser 1 cm) gefüllt. Der Auslauf einer Glassäule (Durchmesser 1 cm) die 0,1 ml immobilisierte Trypsin-Agarosegel (TAG) enthielt, wurde durch eine direkte Schlauchverbindung mit dem Einlauf der APCHS-Säule verbunden. Über ein Ventil und eine Pumpe konnte der Auslauf der APCHS-Säule mit dem Einlauf der TAG-Säule verbunden werden, wodurch ein Flüssigkeitskreislauf entsteht. Beide Säulen wurden mit 20 mM Tris/HCl-Puffer, pH 8,0, equilibriert.

rFII wurde zu 0,4 mg/ml (Aktivität 3,5 I.E./ml) in 4 ml 20 mM Tris/HCl-Puffer, pH 8,0, gelöst und mit einer Fließgeschwindigkeit von 0,8 ml/min durch die TAG-Säule gepumpt. Von dort wurde der Flüssigkeitsstrom ohne Unterbrechung direkt auf die APCHS-Säule geführt und nach der Passage der APCHS-Säule ein zweites Mal mittels Pumpe wiederum durch die TAG-Säule und APCHS-Säule gepumpt. Anschließend wurde die APCHS-Säule von der TAG-Säule getrennt, mit 50 mM Citratpuffer, pH 6,5, gespült (wobei das nicht-gebundene Material entfernt wurde) und dann mit 50 mM Na-Citratpuffer, pH 6,5, 500 mM NaCl, gewaschen (0,5 M NaCl-Eluat). Anschließend wurde die APCHS-Säule mit 1,5 M KSCN in 50 mM Citratpuffer, pH 6,5, eluiert (1,5 M KSCN-Eluat). Die während der Aktivierung erhaltenen Fraktionen wurden auf Thrombinaktivität (I.E./ml), Gesamtaktivität (I.E.) und spezifische Aktivität (Thrombinaktivität/mg Protein) untersucht. Die Ergebnisse der rFII-Aktivierung sind in Tabelle 4 zusammengestellt.

**TABELLE 4**

| Probe | rFIIa Aktivität | | rFIIa spezifische Aktivität |
|---|---|---|---|
| | (I.E./ml) | (I.E. gesamt) | (I.E./mg Protein) |
| Ausgangmaterial | 0 | 0 | 0 |
| Nicht-gebundenes Material | 0 | 0 | 0 |
| 0,5 M NaCl-Eluat | 55 | 110 | 280 |
| 1,5 M KSCN-Eluat | 320 | 2400 | 3500 |

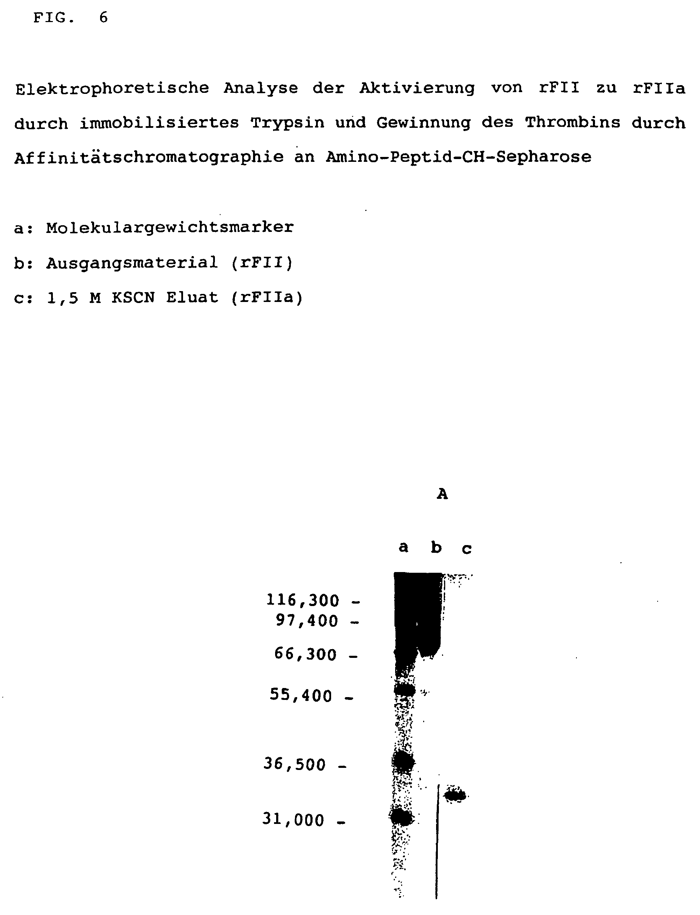

In Fig.6 ist die elektrophoretische Analyse der Aktivierung von rFII zu rFIIa durch immobilisiertes Trypsin und Gewinnung des Thrombins durch Affinitätschromatographie an Amino-Peptid-CH-Sepharose dargestellt, wobei in Bahn a der Molekulargewichtsmarker, in Bahn b das Ausgangsmaterial (rFII) und in Bahn c das 1,5 M KSCN-Eluat (rFIIa) aufgetrennt worden sind.

Die Ergebnisse zeigen, daß durch das im Beispiel 5 beschriebene Verfahren rFII effektiv in rFIIa umgewandelt wurde. Gebildeter rFIIa akkumulierte an der APCHS-Säule und wurde in elektrophoretisch reiner Form mit einem Molekulargewicht von 33000 und mit sehr hoher spezifischer Aktivität durch spezifische Elution der APCHS-Säule erhalten. Durch das beschriebene Verfahren konnten aus 1 I.E. rFII mehr als 150 I.E. reiner rFIIa gewonnen werden.

### Beispiel 6

### Aktivierung von rekombinantem Prothrombin zu Thrombin durch immobilisiertes Trypsin und Gewinnung des Thrombins durch Affinitatschromatographie an Benzamidin-Sepharose.

2 ml Benzamidin-Sepharose (BAS, Firma Pharmacia) wurde in eine Glassäule (Durchmesser 1 cm) gefüllt. Der Auslauf einer Glassäule (Durchmesser 1 cm), die 0,1 ml immobilisierte Trypsin-Agarosegel (TAG) enthielt, wurde durch eine direkte Schlauchverbindung mit dem Einlauf der BAS-Säule verbunden. Über ein Ventil und eine Pumpe konnte der Auslauf der BAS-Säule mit dem Einlauf der TAG-Säule verbunden werden, wodurch ein Flüssigkeitskreislauf entsteht. Beide Säulen wurden mit 20 mM Tris/HCl-Puffer, pH 8,0, equilibriert.

rFII wurde zu 0,4 mg/ml (Aktivität 3,5 I.E./ml) in 4 ml 20 mM Tris/HCl-Puffer, pH 8,0, gelöst und mit einer Fließgeschwindigkeit von 0,8 ml/min durch die TAG-Säule gepumpt. Von dort wurde der Flüssigkeitsstrom ohne Unterbrechung direkt auf die BAS-Säule geführt und nach der Passage der BAS-Säule ein zweites Mal mittels Pumpe wiederum durch die TAG-Säule und BAS-Säule gepumpt. Anschließend wurde die BAS-Säule von der TAG-Säule getrennt, mit 50 mM Citratpuffer, pH 6,5 gespült (um nicht-gebundenes Material zu entfernen) und dann mit 50 mM Na-Citratpuffer, pH 6,5, 150 mM NaCl, gewaschen (0,15 M NaCl-Eluat). Anschließend wurde die BAS-Säule mit 0,1 M Benzamidin in 50 mM Citratpuffer, pH 6,5, eluiert (0,1 M Benzamidin-Eluat). Die während der Aktivierung erhaltenen Fraktionen wurden auf Thrombinaktivität (I.E./ml), Gesamtaktivität (I.E.) und spezifische Aktivität (Thrombinaktivität/mg Protein) untersucht. Die Ergebnisse der rFII-Aktivierung sind in Tabelle 5 aufgelistet.

**TABELLE 5**

| Probe | rFIIa Aktivität | | rFIIa spezifische Aktivität |
|---|---|---|---|
| | (I.E./ml) | (I.E. gesamt) | (I.E./mg Protein) |
| Ausgangmaterial | 0 | 0 | 0 |
| Nicht-gebundenes Material | 0 | 0 | 0 |
| 0,15 M NaCl-Eluat | 0 | 0 | 0 |
| 0,1 M Benzamidin-Eluat | 670 | 2100 | 360 |

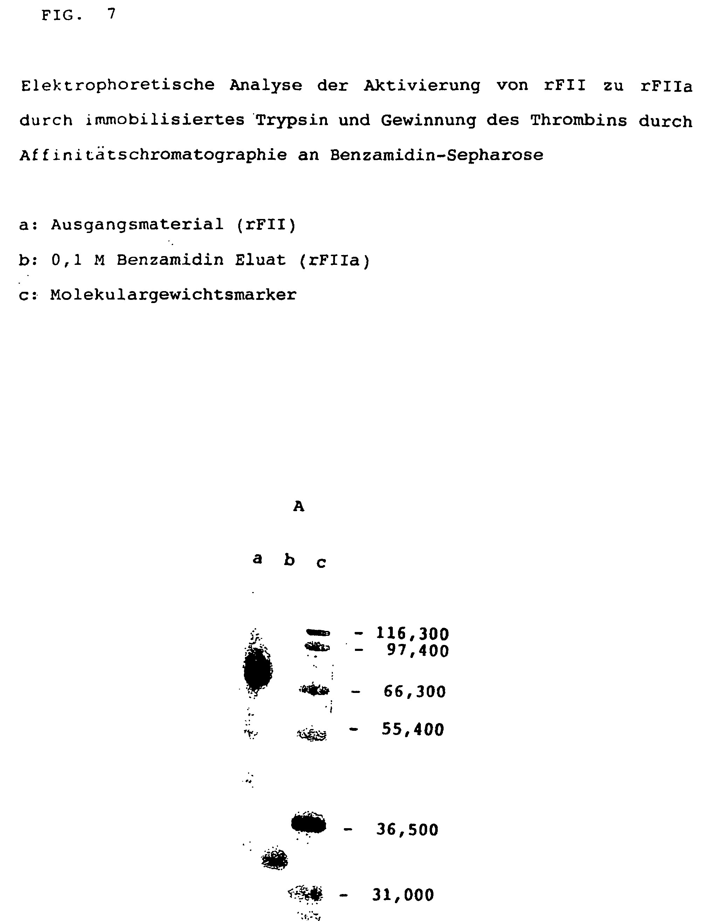

Fig. 7 zeigt die elektrophoretische Analyse der Aktivierung von rFII zu rFIIa durch immobilisiertes Trypsin und Gewinnung des Thrombins durch Affinitätschromatographie an Benzamidin-Sepharose, wobei in Bahn a das Ausgangsmaterial (rFII), in Bahn b das 0,1 M Benzamidin-Eluat (rFIIa) und in Bahn c der Molekulargewichtsmarker aufgetragen worden sind.

Die Ergebnisse zeigen, daß durch das im Beispiel 6 beschriebene Verfahren rFII effektiv in rFIIa umgewandelt wurde. Gebildeter rFIIa akkumulierte an der BAS-Säule und wurde in elektrophoretisch reiner Form mit einem Molekulargewicht von 33000 und mit sehr hoher spezifischer Aktivität durch spezifische Elution der BAS-Säule erhalten. Durch das beschriebene Verfahren konnten aus 1 I.E. rFII mehr als 150 I.E. reiner rFIIa gewonnen werden.

### Beispiel 7

### Aktivierung von rekombinantem Prothrombin zu Thrombin durch immobilisiertes Trypsin und Gewinnung des Thrombins durch Affinitätschromatographie an Heparin-Sepharose.

2 ml Heparin-Sepharose Fast Flow (HS, Pharmacia) wurde in eine Glassäule (Durchmesser 1 cm) gefüllt. Der Auslauf einer Glassäule (Durchmesser 1 cm), die 0,1 ml immobilisierte Trypsin-Agarosegel (TAG) enthielt, wurde durch eine direkte Schlauchverbindung mit dem Einlauf der HS-Säule verbunden. Über ein Ventil und eine Pumpe konnte der Auslauf der BAS-Säule mit dem Einlauf der TAG-Säule verbunden werden, wodurch ein Flüssigkeitskreislauf entsteht. Beide Säulen wurden mit 20 mM Tris/HCl-Puffer, pH 8,0, equilibriert. rFII wurde zu 0,4 mg/ml (Aktivität 3,5 I.E./ml) in 4 ml 20 mM Tris/HCl-Puffer, pH 8,0, gelöst und mit einer Fließgeschwindigkeit von 0,8 ml/min durch die TAG-Säule gepumpt. Von dort wurde der Flüssigkeitsstrom ohne Unterbrechung direkt auf die HS-Säule geführt und nach der Passage der HS-Säule ein zweites Mal mittels Pumpe wiederum durch die TAG-Säule und HS-Säule gepumpt. Anschließend wurde die HS-Säule von der TAG-Säule getrennt, mit 50 mM Citratpuffer, pH 6,5, gespült (um nicht-gebundenes Material zu entfernen) und dann mit 50 mM Na-Citratpuffer, pH 6,5, 150 mM NaCl, gewaschen (0,15 M NaCl-Eluat). Anschließend wurde die HS-Säule mit 0,5 M NaCl in 50 mM Citratpuffer, pH 6,5, eluiert (0,5 M NaCl-Eluat). Die während der Aktivierung erhaltenen Fraktionen wurden auf Thrombinaktivität (I.E./ml), Gesamtaktivität (I.E.) und spezifische Aktivität (Thrombinaktivität/mg Protein) untersucht. In Tabelle 6 sind die Ergebnisse der rFII-Aktivierung aus Beispiel 7 zusammengestellt.

**TABELLE 6**

| Probe | rFIIa Aktivität | | rFIIa spezifische Aktivität |
|---|---|---|---|
| | (I.E./ml) | (I.E. gesamt) | (I.E./mg Protein) |
| Ausgangmaterial | 0 | 0 | 0 |
| Nicht-gebundenes Material | 1 | 10 | 5 |
| 0,15 M NaCl-Eluat | 0 | 0 | 0 |
| 0,5 M NaCl-Eluat | 400 | 2110 | 2670 |

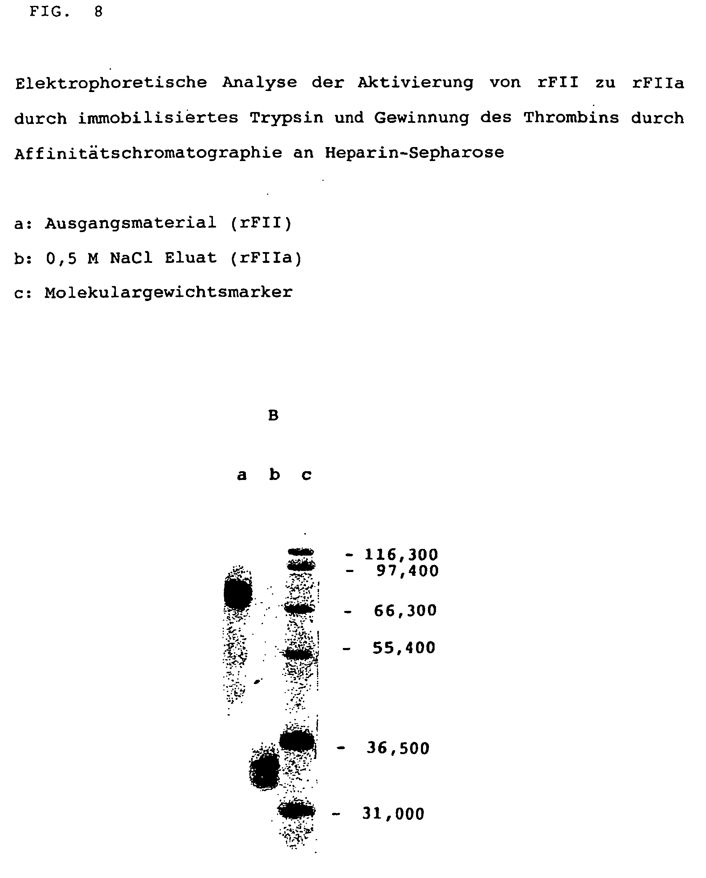

Die elektrophoretische Analyse der Aktivierung von rFII zu rFIIa durch immobilisiertes Trypsin und Gewinnung des Thrombins durch Affinitätschromatographie an Heparin-Sepharose ist in Fig. 8 gezeigt, wobei in Bahn a das Ausgangsmaterial (rFII), in Bahn b das 0,5 M NaCl-Eluat (rFIIa) und in Bahn c der Molekulargewichtsmarker aufgetragen worden sind.

Die Ergebnisse zeigen, daß durch das im Beispiel 7 beschriebene Verfahren rFII effektiv in rFIIa umgewandelt wurde. Gebildeter rFIIa akkumulierte an der HS-Säule und wurde in elektrophoretisch reiner Form mit Molekulargewichten von 33000 und 35000 und mit sehr hoher spezifischer Aktivität durch spezifische Elution der HS-Säule erhalten. Durch das beschriebene Verfahren konnten aus 1 I.E. rFII mehr als 150 I.E. reiner rFIIa gewonnen werden.

### Beispiel 8

### Aktivierung von rekombinantem Prothrombin zu Thrombin in einer Proteinmischung durch immobilisiertes Trypsin und Gewinnung des Thrombins durch Affinitätschromatographie an Amino-Peptid-CH-Sepharose.

Eine Amino-Peptid-CH-Sepharose-Säule (APCHS-Säule) wurde wie im Beispiel 5 beschrieben hergestellt. Der Auslauf einer Glassäule (Durchmesser 1 cm) die 0,1 ml immobilisierte Trypsin-Agarosegel (TAG) enthielt, wurde durch eine direkte Schlauchverbindung mit dem Einlauf der APCHS-Säule verbunden. Über ein Ventil und eine Pumpe konnte der Auslauf der APCHS-Säule mit dem Einlauf der TAG-Säule verbunden werden, wodurch ein Flüssigkeitskreislauf entsteht. Beide Säulen wurden mit 20 mM Tris/HCl-Puffer, pH 8,0, equilibriert.

4 ml eines Proteingemisches (Proteinkonzentration 1,7 mg/ml), welches rFII enthielt (Aktivität 3,5 I.E./ml), in 20 mM Tris/-HCl-Puffer, pH 8,0, wurde mit einer Fließgeschwindigkeit von 0,8 ml/min durch die TAG-Säule gepumpt. Von dort wurde der Flüssigkeitsstrom ohne Unterbrechung direkt auf die APCHS-Säule geführt und nach der Passage der APCHS-Säule ein zweites Mal mittels Pumpe wiederum durch die TAG-Säule und APCHS-Säule gepumpt. Anschließend wurde die APCHS-Säule von der TAG-Säule getrennt, mit 50 mM Citratpuffer, pH 6,5, gespült (zur Entfernung von nicht-gebundenem Material) und dann mit 50 mM Na-Citratpuffer, pH 6,5, 500 mM NaCl, gewaschen (0,5 M NaCl-Eluat). Anschließend wurde die APCHS-Säule mit 1,5 M KSCN in 50 mM Citratpuffer, pH 6,5, eluiert (1,5 M KSCN-Eluat). Die während der Aktivierung erhaltenen Fraktionen wurden auf Thrombinaktivität (I.E./ml), Gesamtaktivität (I.E.) und pezifische Aktivität (Thrombinaktivität/mg Protein) untersucht. In Tabelle 7 sind die Ergebnisse der rFII-Aktivierung aus Beispiel 8 zusammengestellt.

**TABELLE 7**

| Probe | rFIIa Aktivität | | rFIIa spezifische Aktivität |
|---|---|---|---|
| | (I.E./ml) | (I.E. gesamt) | (I.E./mg Protein) |
| Ausgangmaterial | 0 | 0 | 0 |
| Nicht-gebundenes Material | 0 | 0 | 0 |
| 0,5 M NaCl-Eluat | 44 | 120 | 230 |
| 1,5 M KSCN-Eluat | 305 | 2250 | 3300 |

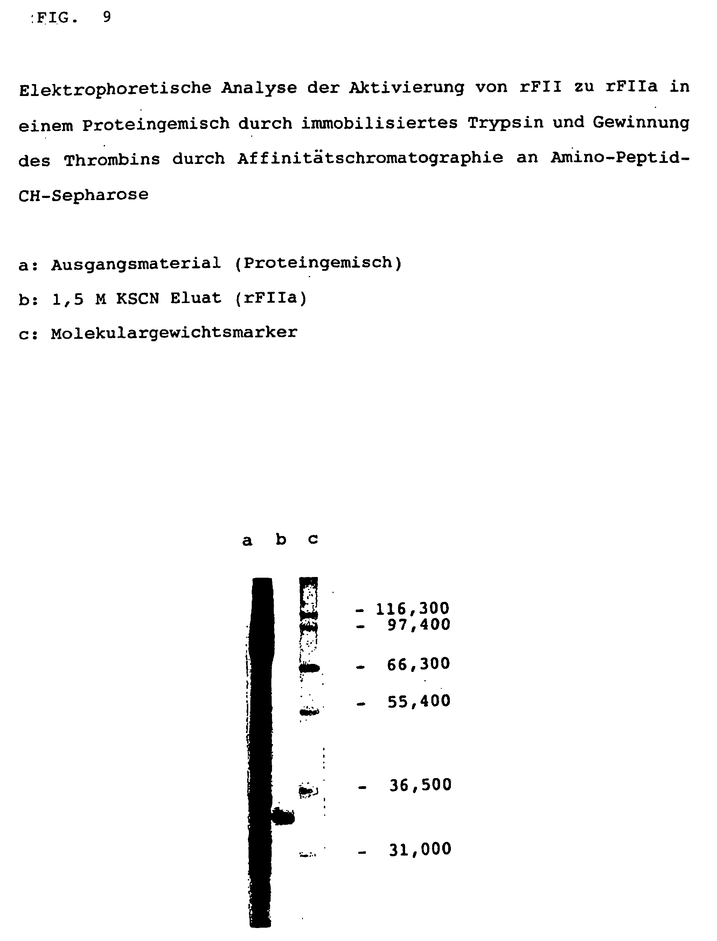

In Fig. 9 ist die elektrophoretische Analyse der Aktivierung von rFII zu rFIIa in einem Proteingemisch durch immobilisiertes Trypsin und Gewinnung des Thrombins durch Affinitätschromatographie an Amino-Peptid-CH-Sepharose gezeigt, wobei in Bahn a das Ausgangsmaterial (Proteingemisch), in Bahn b das 1,5 M KSCN-Eluat (rFIIa) und in Bahn c der Molekulargewichtsmarker aufgetragen worden sind.

Die Ergebnisse zeigen, daß durch das im Beispiel 8 beschriebene Verfahren rFII in einem Proteingemisch effektiv in rFIIa umgewandelt wurde. Gebildeter rFIIa akkumulierte an der APCHS-Säule und wurde in elektrophoretisch reiner Form mit einem Molekulargewicht von 33000 und mit sehr hoher spezifischer Aktivität durch spezifische Elution der APCHS-Säule erhalten. Durch das beschriebene Verfahren konnten aus 1 I.E. rFII mehr als 150 I.E. reiner rFIIa gewonnen werden.

### Beispiel 9

### Aktivierung von humanem Prothrombin zu Thrombin durch immobilisiertes Trypsin und Gewinnung des Thrombins durch Affinitätschromatographie an Benzamidin-Sepharose.

2 ml Benzamidin-Sepharose (BAS, Pharmacia) wurde in eine Glassäule (Durchmesser 1 cm) gefüllt. Der Auslauf einer Glassäule (Durchmesser 1 cm), die 0,1 ml immobilisierte Trypsin-Agarosegel (TAG) enthielt, wurde durch eine direkte Schlauchverbindung mit dem Einlauf der BAS-Säule verbunden. Über ein Ventil und eine Pumpe konnte der Auslauf der BAS-Säule mit dem Einlauf der TAG-Säule verbunden werden, wodurch ein Flüssigkeitskreislauf entsteht. Beide Säulen wurden mit 20 mM Tris/HCl-Puffer, pH 8,0, equilibriert.

Humaner Faktor II (hFII) wurde zu 0,5 mg/ml (Aktivität 4 I.E./ml) in 4 ml 20 mM Tris/HCl-Puffer, pH 8,0, gelöst und mit einer Fließgeschwindigkeit von 0,8 ml/min durch die TAG-Säule gepumpt. Von dort wurde der Flüssigkeitsstrom ohne Unterbrechung direkt auf die BAS-Säule geführt und nach der Passage der BAS-Säule ein zweites Mal mittels Pumpe wiederum durch die TAG-Säule und BAS-Säule gepumpt. Anschließend wurde die BAS-Säule von der TAG-Säule getrennt, mit 50 mM Citratpuffer, pH 6,5, gespült (dabei wurde nicht-gebundenes Material entfernt) und dann mit 50 mM Na-Citratpuffer, pH 6,5, 150 mM NaCl, gewaschen (0,15 M NaCl-Eluat). Anschließend wurde die BAS-Säule mit 0,1 M Benzamidin in 50 mM Citratpuffer, pH 6,5, eluiert (0,1 M Benzamidin-Eluat). Die während der Aktivierung erhaltenen Fraktionen wurden auf Thrombinaktivität (I.E./ml), Gesamtaktivität (I.E.) und spezifische Aktivität (Thrombinaktivität/mg Protein) untersucht. Die Ergebnisse der FII-Aktivierung aus Beispiel 9 sind in Tabelle 8 zusammenfassend dargestellt.

**TABELLE 8**

| Probe | rFIIa Aktivität | | rFIIa spezifische Aktivität |
|---|---|---|---|
| | (I.E./ml) | (I.E. gesamt) | (I.E./mg Protein) |
| Ausgangmaterial | 0 | 0 | 0 |
| Nicht-gebundenes Material | 0,5 | 3 | 1,7 |
| 0,15 M NaCl-Eluat | 0 | 0 | 0 |
| 0,1 M Benzamidin-Eluat | 491 | 2450 | 3270 |

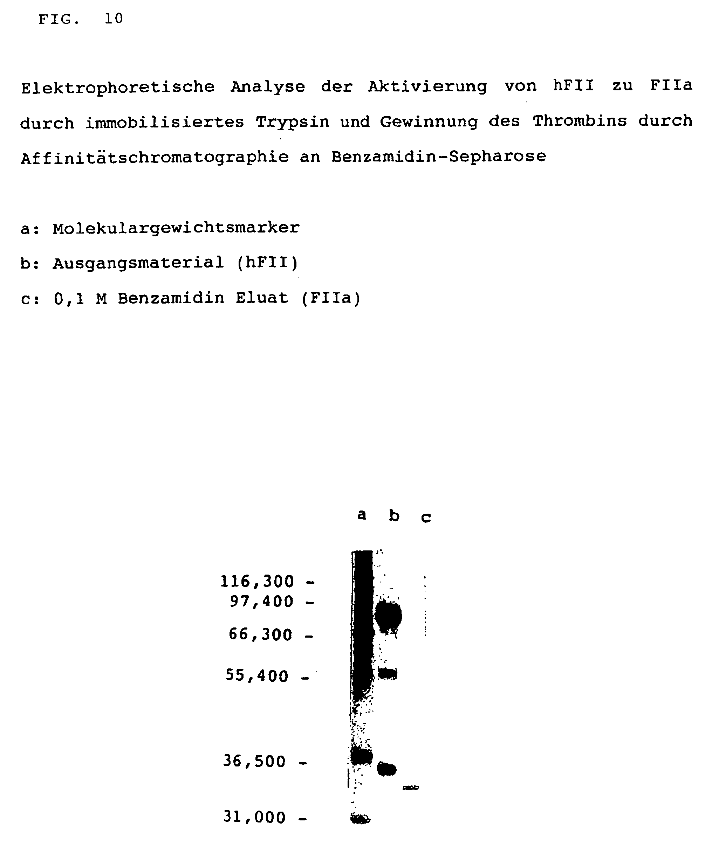

Fig. 10 zeigt die elektrophoretische Analyse der Aktivierung von hFII zu FIIa durch immobilisiertes Trypsin und Gewinnung des Thrombins durch Affinitätschromatographie an Benzamidin-Sepharose, wobei in Bahn a der Molekulargewichtsmarker, in Bahn b das Ausgangsmaterial (hFII) und in Bahn c das 0,1 M Benzamidin-Eluat (FIIa) aufgetragen worden ist.

Die Ergebnisse zeigen, daß durch das im Beispiel 9 beschriebene Verfahren hFII effektiv in FIIa umgewandelt wurde. Gebildeter FIIa akkumulierte an der BAS-Säule und wurde in elektrophoretisch reiner Form mit einem Molekulargewicht von 33000 und mit sehr hoher spezifischer Aktivität durch spezifische Elution der BAS-Säule erhalten. Durch das beschriebene Verfahren konnten aus 1 I.E. FII mehr als 150 I.E. reiner FIIa gewonnen werden.

### Beispiel 10

### Aktivierung von rekombinantem Prothrombin zu Thrombin durch immobilisierten Faktor Xa und Gewinnung des Thrombins durch Affinitätschromatographie an Amino-Peptid-CH-Sepharose.

Eine Amino-Peptid-CH-Sepharose-Säule (APCHS-Säule) wurde wie im Beispiel 5 beschrieben hergestellt. 30 mg der Protease Faktor Xa (Boehringer Mannheim) wurde entsprechend der Herstellervorschrift an 1 ml CNBr-aktivierte Sepharose (Pharmacia) gekoppelt und in eine Glassäule (Durchmesser 1 cm) gefüllt (XaS-Säule). Der Auslauf der XaS-Säule wurde durch eine direkte Schlauchverbindung mit dem Einlauf der APCHS-Säule verbunden. Über ein Ventil und eine Pumpe konnte der Auslauf der APCHS-Säule mit dem Einlauf der XaS-Säule verbunden werden, wodurch ein Flüssigkeitskreislauf entsteht. Beide Säulen wurden mit 20 mM Tris/HCl-Puffer, pH 8,0, equilibriert.

rFII wurde zu 0,4 mg/ml (Aktivität 3,5 I.E./ml) in 4 ml 20 mM Tris/HCl-Puffer, pH 8,0, gelöst und mit einer Fließgeschwindigkeit von 0,1 ml/min durch die XaS-Säule gepumpt. Von dort wurde der Flüssigkeitsstrom ohne Unterbrechung direkt auf die APCHS-Säule geführt und nach der Passage der APCHS-Säule ein zweites Mal mittels Pumpe wiederum durch die XaS-Säule und APCHS-Säule gepumpt. Dieser Vorgang wurde noch drei Mal wiederholt. Anschließend wurde die APCHS-Säule von der XaS-Säule getrennt, mit 50 mM Citratpuffer, pH 6,5, gespült (wobei nicht-gebundenes Material entfernt wurde) und dann mit 50 mM Na-Citratpuffer, pH 6,5, 500 mM NaCl, gewaschen (0,5 M NaCl-Eluat). Anschließend wurde die APCHS-Säule mit 1,5 M KSCN in 50 mM Citratpuffer, pH 6,5, eluiert (1,5 M KSCN-Eluat). Die während der Aktivierung erhaltenen Fraktionen wurden auf Thrombinaktivität (I.E./ml), Gesamtaktivität (I.E.) und spezifische Aktivität (Thrombinaktivität/mg Protein) untersucht. In Tabelle 9 sind die Ergebnisse der rFII-Aktivierung aus Beispiel 10 zusammengefaßt.

**TABELLE 9**

| Probe | rFIIa Aktivität | | rFIIa spezifische Aktivität |
|---|---|---|---|
| | (I.E./ml) | (I.E. gesamt) | (I.E./mg Protein) |
| Ausgangmaterial | 0 | 0 | 0 |
| Nicht-gebundenes Material | 0 | 0 | 0 |
| 0,5 M NaCl-Eluat | 35 | 80 | 80 |
| 0,1 M KSCN-Eluat | 30 | 2230 | 3050 |

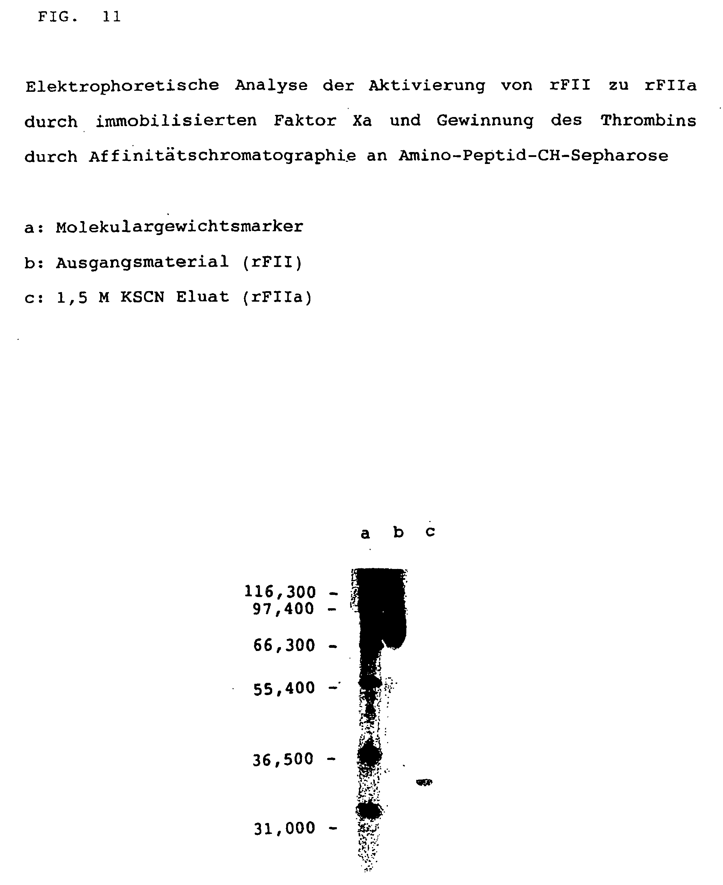

Fig. 11 zeigt die elektrophoretische Analyse der Aktivierung von rFII zu rFIIa durch immobilisierten Faktor Xa und Gewinnung des Thrombins durch Affinitätschromatographie an Amino-Peptid-CH-Sepharose, wobei in Bahn a der Molekulargewichtsmarker, in Bahn b das Ausgangsmaterial (rFII) und in Bahn c das 1,5 M KSCN-Eluat (rFIIa) aufgetragen worden ist.

Die Ergebnisse zeigen, daß durch das im Beispiel 10 beschriebene Verfahren rFII auch durch die Protease Faktor Xa effektiv in rFIIa umgewandelt wurde. Gebildeter rFIIa akkumulierte an der APCHS-Säule und wurde in elektrophoretisch reiner Form mit einem Molekulargewicht von 33000 und mit sehr hoher spezifischer Aktivität durch spezifische Elution der APCHS-Säule erhalten. Durch das beschriebene Verfahren konnten aus 1 I.E. rFII mehr als 150 I.E. reiner rFIIa gewonnen werden.

### Beispiel 11

### Aktivierung von Faktor X zu Faktor Xa durch immobilisiertes Trypsin und Gewinnung des Faktors Xa durch Affinitätschromatographie an Benzamidin-Sepharose.

2 ml Benzamidin-Sepharose (BAS, Pharmacia) wurden in eine Glassäule (Durchmesser 1 cm) gefüllt. Der Auslauf einer Glassäule (Durchmesser 1 cm), die 0,1 ml immobilisiertes Trypsin-Agarosegel (TAG) enthielt, wurde durch eine direkte Schlauchverbindung mit dem Einlauf der BAS-Säule verbunden. Über ein Ventil und eine Pumpe konnte der Auslauf der BAS-Säule mit dem Einlauf der TAG-Säule verbunden werden, wodurch ein Flüssigkeitskreislauf entsteht. Beide Säulen wurden mit 20 mM Tris/HCl-Puffer, pH 8,0, equilibriert.

2 ml einer Lösung von Faktor X (FX, Firma Boehringer Mannheim) wurden zu 0,5 mg/ml in 20 mM Tris/HCl-Puffer, pH 8,0, gelöst und mit einer Fließgeschwindigkeit von 0,5 ml/min durch die TAG-Säule gepumpt. Von dort wurde der Flüssigkeitsstrom ohne Unterbrechung direkt auf die BAS-Säule geführt und nach der Passage der BAS-Säule ein zweites Mal mittels Pumpe wiederum durch die TAG-Säule und BAS-Säule gepumpt. Anschließend wurde die BAS-Säule von der TAG-Säule getrennt, mit 50 mM Citratpuffer, pH 6,5, gespült (Entfernen von nicht-gebundenem Material) und dann mit 50 mM Na-Citratpuffer, pH 6,5, 150 mM NaCl, gewaschen (0,15 M NaCl-Eluat). Anschließend wurde die BAS-Säule mit 0,1 M Benzamidin in 50 mM Citratpuffer, pH 6,5, eluiert (0,1 M Benzamidin-Eluat). Die während der Aktivierung erhaltenen Fraktionen wurden auf Aktivität des Faktors Xa (I.E./ml), Gesamtaktivität (I.E.) und spezifische Aktivität (Aktivität/mg Protein) untersucht. Die Ergebnisse der Faktor X-Aktivierung aus Beispiel 11 sind in Tabelle 10 zusammengefaßt.

**TABELLE 10**

| Probe | FXa Aktivität | | FXa spezifische Aktivität |
|---|---|---|---|
| | (I.E./ml) | (I.E. gesamt) | (I.E./mg Protein) |
| Ausgangmaterial | 0 | 0 | 0 |
| Nicht-gebundenes Material | 20 | 100 | 30 |
| 0,15 M NaCl-Eluat | 0 | 0 | 0 |
| 0,1 M Benzamidin-Eluat | 520 | 1560 | 2600 |

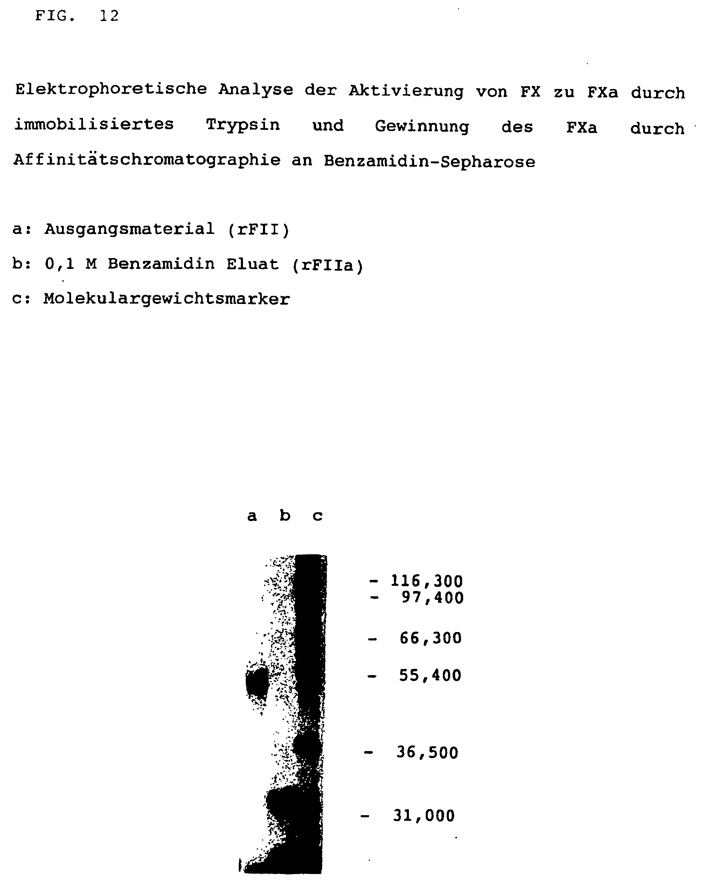

Fig. 12 zeigt die elektrophoretische Analyse der Aktivierung von FX zu FXa durch immobilisiertes Trypsin und Gewinnung des FXa durch Affinitätschromatographie an Benzamidin-Sepharose, wobei in Bahn a das Ausgangsmaterial (FX), in Bahn b das 0,1 M Benzamidin-Eluat (FXa) und in Bahn c der Molekulargewichtsmarker aufgetragen worden sind.

Die Ergebnisse zeigen, daß durch das im Beispiel 11 beschriebene Verfahren FX effektiv in FXa umgewandelt wurde. Gebildeter FXa akkumulierte an der BAS-Säule und wurde in elektrophoretisch reiner Form mit einem Molekulargewicht von 32000 und mit sehr hoher spezifischer Aktivität durch spezifische Elution der BAS-Säule erhalten.

## Patentansprüche

1. Verfahren zur Herstellung und Gewinnung von Proteinen aus Pro-Proteinen in einem Verfahrensschritt, **dadurch gekennzeichnet, daß** eine Pro-Protein-haltige Lösung mit einer Protease und einem festen Träger, der eine höhere Affinität zum Protein als zum Pro-Protein oder dessen funktionell inaktiven Abbauprodukten aufweist, in Kontakt gebracht wird, wobei das Pro-Protein zum Protein proteolytisch gespalten wird und das Protein durch Adsorption an den festen Träger selektiv abgetrennt wird.

2. Verfahren nach Anspruch 1, **dadurch gekennzeichnet, daß** die Protease immobilisiert ist.

3. Verfahren nach Anspruch 1 oder 2, **dadurch gekennzeichnet, daß** die Pro-Protein-haltige Lösung mit der Protease während einer gegebenen Kontaktdauer behandelt wird, die ausreicht, um eine Spaltung des Pro-Proteins zum Protein herbeizuführen, bei welcher im wesentlichen keine weiteren funktionell inaktiven Abbauprodukte des Proteins gebildet werden.

4. Verfahren nach Anspruch 1 bis 3, **dadurch gekennzeichnet, daß** nach dem Kontakt mit der Protease und Abtrennung des Proteins gegebenenfalls in Lösung verbliebenes, nicht-gespaltenes Pro-Protein erneut mit der Protease in Kontakt gebracht, das gebildete Protein an den festen Träger adsorbiert wird und diese Schritte gegebenenfalls solange wiederholt werden, bis im wesentlichen das gesamte Pro-Protein zu Protein gespalten worden ist.

5. Verfahren nach Anspruch 1 oder 4, **dadurch gekennzeichnet, daß** der (die) Protasebehandlungsschritt(e) und der (die) Adsorptionsschritt(e) kontinuierlich durchgeführt werden.

6. Verfahren nach einem der Ansprüche 1 bis 5, **dadurch gekennzeichnet, daß** das Pro-Protein ein Proenzym und das Protein ein Enzym ist.

7. Verfahren nach einem der Ansprüche 1 bis 6, **dadurch gekennzeichnet, daß** die Kontaktdauer von Pro-Protein und Protease maximal 24 Stunden beträgt.

8. Verfahren nach einem der Ansprüche 1 bis 7, **dadurch gekennzeichnet, daß** die Kontaktdauer von Pro-Protein und Protease zwischen 1 Sekunde und 24 Stunden, vorzugsweise zwischen 1 Sekunde und 5 Stunden, besonders bevorzugt zwischen 1 Sekunde und 30 Minuten, liegt.

9. Verfahren nach einem der Ansprüche 1 bis 8, **dadurch gekennzeichnet, daß** die Protease aus Trypsin, Chymotrypsin, Faktor Xa, Venom-Protease, Thrombin, Plasmin oder einer Serin-Protease der Subtilisin-Familie, insbesondere Kexin- oder Furin-artige Proteasen, ausgewählt ist.

10. Verfahren nach einem der Ansprüche 1 bis 9, **dadurch gekennzeichnet, daß** das Pro-Protein eine inaktive Vorstufe eines Blutgerinnungsfaktors, vorzugsweise Faktor II, Faktor V, Faktor VII, Faktor VIII, Pro-Faktor IX, Faktor IX, Faktor X, Faktor XIII, Protein C oder von Willebrand-Faktor, und das Protein das funktionell aktive Gerinnungsprotein ist.

11. Verfahren nach einem der Ansprüche 1 bis 10, **dadurch gekennzeichnet, daß** als Pro-Protein-haltige Lösung eine Lösung eingesetzt wird, die das Pro-Protein in gereinigter Form enthält.

12. Verfahren nach einem der Ansprüche 1 bis 11, **dadurch gekennzeichnet, daß** als Pro-Protein-haltige Lösung eine Lösung eingesetzt wird, in der das Protein in Mischung mit anderen Proteinen vorliegt.

13. Verfahren nach einem der Ansprüche 1 bis 10 und 12, **dadurch gekennzeichnet, daß** als Pro-Protein-haltige Lösung eine Lösung eingesetzt wird, die biologischen Ursprungs ist.

14. Verfahren nach einem der Ansprüche 1 bis 13, **dadurch gekennzeichnet, daß** als Pro-Protein-haltige Lösung Plasma, eine Plasmafraktion oder eine davon abgeleitete Lösung eingesetzt wird.

15. Verfahren nach einem der Ansprüche 1 bis 12, **dadurch gekennzeichnet, daß** als Pro-Protein-haltige Lösung eine durch biotechnologische Verfahren hergestellte Lösung eingesetzt wird.

16. Verfahren nach Anspruch 15, **dadurch gekennzeichnet, daß** als Pro-Protein-haltige Lösung ein von einer rekombinanten Zellkultur erzeugter Kulturüberstand eingesetzt wird.

17. Verfahren nach einem der Ansprüche 1 bis 16, **dadurch gekennzeichnet, daß** der feste Träger ausgewählt ist aus immobilisiertem Hirudin oder von Hirudin abgeleiteten Polypeptiden oder Derivaten, immobilisiertem Heparin, immobilisiertem Benzamidin, und immobilisierten Proteinen oder Peptiden, insbesondere immobilisierten Antikörpern.

18. Verfahren nach einem der Ansprüche 1 bis 17, **dadurch gekennzeichnet, daß** das am festen Träger adsorbierte Protein vom Träger selektiv eluiert wird.

19. Verwendung eines Verfahrens nach einem der Ansprüche 1 bis 18 zur Herstellung von Proteinen aus Pro-Proteinen.

20. Verwendung eines Verfahrens nach einem der Ansprüche 1 bis 18, zur Herstellung von aktivierten Enzymen aus Pro-Enzymen.

21. Verwendung eines Verfahrens nach einem der Ansprüche 1 bis 18, zur Herstellung von aktiviertem Faktor II.

22. Verwendung eines Verfahrens nach einem der Ansprüche 1 bis 18, zur Herstellung von aktiviertem Faktor V.

23. Verwendung eines Verfahrens nach einem der Ansprüche 1 bis 18, zur Herstellung von aktiviertem Faktor VII.

24. Verwendung eines Verfahrens nach einem der Ansprüche 1 bis 18, zur Herstellung von aktiviertem Faktor VIII.

25. Verwendung eines Verfahrens nach einem der Ansprüche 1 bis 18, zur Herstellung von Faktor IX oder aktiviertem Faktor IX.

26. Verwendung eines Verfahrens nach einem der Ansprüche 1 bis 18, zur Herstellung von aktiviertem Faktor X.

27. Verwendung eines Verfahrens nach einem der Ansprüche 1 bis 18, zur Herstellung von aktiviertem Faktor XIII.

28. Verwendung eines Verfahrens nach einem der Ansprüche 1 bis 18, zur Herstellung von aktiviertem Protein C.

29. Verwendung eines Verfahrens nach einem der Ansprüche 1 bis 18, zur Herstellung von von Willebrand-Faktor.

30. Verwendung von an einem festen Träger immobilisierten Hirudin oder Hirudin-Fragmenten oder Hirudin-Derivaten in einem Verfahren gemäß einem der Ansprüche 1 bis 18.

## Claims

1. A method of preparing and recovering proteins from pro-proteins in one method step, **characterised in that** a pro-protein-containing solution is contacted with a protease and with a solid carrier having a higher affinity to the protein than to the pro-protein or to the functionally inactive degradation products thereof, wherein the pro-protein is proteolytically cleaved to the protein, and the protein is selectively separated by adsorption on the solid carrier.

2. A method according to claim 1, **characterised in that** the protease is immobilized.

3. A method according to claim 1 or 2, **characterised in that** the pro-protein-containing solution is treated with the protease for a given period of contact which suffices to induce a cleavage of the pro-protein to protein, in which substantially no further functionally inactive degradation products of the protein are formed.

4. A method according to claims 1 to 3, **characterised in that** after the contact with the protease and the separation of the protein, possibly non-cleaved pro-protein that has remained in solution is again contacted with the protease, the protein formed is adsorbed on the solid carrier, and these steps optionally are repeated until substantially the entire pro-protein has been cleaved to protein.

5. A method according to claim 1 or 4, **characterised in that** the protease treatment step(s) and the adsorption step(s) are carried out continuously.

6. A method according to any one of claims 1 to 5, **characterised in that** the pro-protein is a pro-enzyme and the protein is an enzyme.

7. A method according to any one of claims 1 to 6, **characterised in that** the duration of contact of pro-protein and protease is 24 hours at the most.

8. A method according to any one of claims 1 to 7, **characterised in that** the period of contact of pro-protein and protease is between 1 second and 24 hours, preferably between 1 second and 5 hours, particularly preferred between 1 second and 30 minutes.

9. A method according to any one of claims 1 to 8, **characterised in that** the protease is selected from trypsin, chymotrypsin, factor Xa, venom-protease, thrombin, plasmin or a serine-protease of the subtilisin family, in particular kexin-type or furin-type proteases.

10. A method according to any one of claims 1 to 9, **characterised in that** the pro-protein is an inactive pre-stage of a blood clotting factor, preferably factor II, factor V, factor VII, factor VIII, pro-factor IX, factor IX, factor X, factor XIII, protein C or von Willebrand factor, and the protein is the functionally active clotting protein.

11. A method according to any one of claims 1 to 10, **characterised in that** as the pro-protein-containing solution a solution is used which contains the pro-protein in purified form.

12. A method according to any one of claims 1 to 11, **characterised in that** a solution in which the protein is present in admixture with other proteins is used as the pro-protein-containing solution.

13. A method according to any one of claims 1 to 10 and 12, **characterised in that** a solution which is of biological origin is used as the pro-protein-containing solution.

14. A method according to any one of claims 1 to 13, **characterised in that** plasma, a plasma fraction or a solution derived therefrom is used as the pro-protein-containing solution.

15. A method according to any one of claims 1 to 12, **characterised in that** a solution produced by a biotechnological method is used as the pro-protein-containing solution.

16. A method according to claim 15, **characterised in that** a culture supernatant prepared from a recombinant cell culture is used as the pro-protein-containing solution.

17. A method according to any one of claims 1 to 16, **characterised in that** the solid carrier is selected from immobilized hirudin or from polypeptides or derivatives derived from hirudin, immobilized heparin, immobilized benzamidine and immobilized proteins or peptides, in particular immobilized antibodies.

18. A method according to any one of claims 1 to 17, **characterised in that** the protein adsorbed on the solid carrier is selectively eluted from the carrier.

19. The use of a method according to any one of claims 1 to 18 for the preparation of proteins from pro-proteins.

20. The use of a method according to any one of claims 1 to 18 for the preparation of activated enzymes from pro-enzymes.

21. The use of a method according to any one of claims 1 to 18 for the preparation of activated factor II.

22. The use of a method according to any one of claims 1 to 18 for the preparation of activated factor V.

23. The use of a method according to any one of claims 1 to 18 for the preparation of activated factor VII.

24. The use of a method according to any one of claims 1 to 18 for the preparation of activated factor VIII.

25. The use of a method according to any one of claims 1 to 18 for the preparation of factor IX or activated factor IX.

26. The use of a method according to any one of claims 1 to 18 for the preparation of activated factor X.

27. The use of a method according to any one of claims 1 to 18 for the preparation of activated factor XIII.

28. The use of a method according to any one of claims 1 to 18 for the preparation of activated protein C.

29. The use of a method according to any one of claims 1 to 18 for the preparation of von Willebrand factor.

30. The use of hirudin or hirudin fragments or hirudin derivatives immobilized on a solid carrier in a method according to any one of claims 1 to 18.

## Revendications

1. Procédé pour la préparation et l'obtention de protéines à partir de pro-protéines dans une étape de procédé, **caractérisé en ce qu'**une solution contenant une pro-protéine est mise en contact avec une protéase et un support solide qui présente une plus grande affinité pour la protéine que pour la pro-protéine ou ses produits de dégradation fonctionnellement inactifs, où la pro-protéine est clivée protéolytiquement en la protéine et la protéine est séparée sélectivement par adsorption sur le support solide.

2. Procédé selon la revendication 1 **caractérisé en ce que** la protéase est immobilisée.

3. Procédé selon la revendication 1 ou 2 **caractérisé en ce que** la solution contenant une pro-protéine est traitée avec la protéase pendant une durée de contact donnée qui est suffisante pour provoquer un clivage de la pro-protéine en la protéine au cours duquel sensiblement aucun produit de dégradation de la protéine fonctionnellement inactif supplémentaire n'est formé.

4. Procédé selon les revendications 1 à 3 **caractérisé en ce qu'**après le contact avec la protéase et la séparation de la protéine, la pro-protéine non clivée éventuellement restée dans la solution est mise de nouveau en contact avec la protéase, la protéine formée est adsorbée sur le support solide et ces étapes sont éventuellement répétées jusqu'à ce que sensiblement toute la pro-protéine ait été clivée en protéine.

5. Procédé selon la revendication 1 ou 4 **caractérisé en ce que** la ou les étapes de traitement avec une protéase et la ou les étapes d'adsorption sont réalisées en continu.

6. Procédé selon l'une des revendications 1 à 5 **caractérisé en ce que** la pro-protéine est une pro-enzyme et la protéine est une enzyme.

7. Procédé selon l'une des revendications 1 à 6 **caractérisé en ce que** la durée de contact de la pro-protéine et de la protéase est d'au maximum 24 h.

8. Procédé selon l'une des revendications 1 à 7 **caractérisé en ce que** la durée de contact de la pro-protéine et de la protéase est située entre 1 s et 24 h, de préférence entre 1 s et 5 h, de manière particulièrement préférée entre 1 s et 30 min.

9. Procédé selon l'une des revendications 1 à 8 **caractérisé en ce que** la protéase est choisie parmi la trypsine, la chymotrypsine, le facteur Xa, la protéase de venin, la thrombine, la plasmine ou une sérine-protéase de la famille des subtilisines, en particulier les protéases de type kexine ou furine.

10. Procédé selon l'une des revendications 1 à 9 **caractérisé en ce que** la pro-protéine est un précurseur inactif d'un facteur de coagulation sanguine, de préférence le facteur II, le facteur V, le facteur VII, le facteur VIII, le pro-facteur IX, le facteur IX, le facteur X, le facteur XIII, la protéine C ou le facteur de von Willebrand, et la protéine est la protéine de coagulation fonctionnellement active.

11. Procédé selon l'une des revendications 1 à 10 **caractérisé en ce qu'**une solution qui contient la pro-protéine sous forme purifiée est utilisée comme solution contenant une pro-protéine.

12. Procédé selon l'une des revendications 1 à 11 **caractérisé en ce qu'**une solution dans laquelle la protéine est présente en mélange avec d'autres protéines est utilisée comme solution contenant une pro-protéine.

13. Procédé selon l'une des revendications 1 à 10 et 12 **caractérisé en ce qu'**une solution qui est d'origine biologique est utilisée comme solution contenant une pro-protéine.

14. Procédé selon l'une des revendications 1 à 13 **caractérisé en ce que** le plasma, une fraction plasmatique ou une solution qui en est dérivée est utilisé comme solution contenant une pro-protéine.

15. Procédé selon l'une des revendications 1 à 12 **caractérisé en ce qu'**une solution préparée par des procédés biotechnologiques est utilisée comme solution contenant une pro-protéine.

16. Procédé selon la revendication 15 **caractérisé en ce qu'**un surnageant de culture produit par une culture de cellules recombinées est utilisé comme solution contenant une pro-protéine.

17. Procédé selon l'une des revendications 1 à 16 **caractérisé en ce que** le support solide est choisi parmi l'hirudine immobilisée ou les polypeptides ou dérivés issus de l'hirudine, l'héparine immobilisée, la benzamidine immobilisée et les protéines ou peptides immobilisés, en particulier les anticorps immobilisés.

18. Procédé selon l'une des revendications 1 à 17 **caractérisé en ce que** la protéine adsorbée sur le support solide est éluée sélectivement du support.

19. Utilisation d'un procédé selon l'une des revendications 1 à 18 pour la production de protéines à partir de pro-protéines.

20. Utilisation d'un procédé selon l'une des revendications 1 à 18 pour la production d'enzymes activées à partir de pro-enzymes.

21. Utilisation d'un procédé selon l'une des revendications 1 à 18 pour la production de facteur II activé.

22. Utilisation d'un procédé selon l'une des revendications 1 à 18 pour la production de facteur V activé.

23. Utilisation d'un procédé selon l'une des revendications 1 à 18 pour la production de facteur VII activé.

24. Utilisation d'un procédé selon l'une des revendications 1 à 18 pour la production de facteur VIII activé.

25. Utilisation d'un procédé selon l'une des revendications 1 à 18 pour la production de facteur IX ou de facteur IX activé.

26. Utilisation d'un procédé selon l'une des revendications 1 à 18 pour la production de facteur X activé.

27. Utilisation d'un procédé selon l'une des revendications 1 à 18 pour la production de facteur XIII activé.

28. Utilisation d'un procédé selon l'une des revendications 1 à 18 pour la production de protéine C activée.

29. Utilisation d'un procédé selon l'une des revendications 1 à 18 pour la production de facteur de von Willebrand.

30. Utilisation d'hirudine immobilisée sur un support solide ou de fragments d'hirudine ou de dérivés d'hirudine dans un procédé selon l'une des revendications 1 à 18.
